# EUROPEAN PATENT SPECIFICATION

(11) **EP 3 720 467 B1**
(45) Date of publication and mention of the grant of the patent: **24.04.2024**
(21) Application number: 18825584.8
(22) Date of filing: 07.12.2018
(51) Int. Cl.: A61K 38/17, A61P 25/00

(54) **METHODS FOR TREATMENT OF NEUROPATHIC PAIN**
VERFAHREN ZUR BEHANDLUNG VON NEUROPATHISCHEN SCHMERZEN
MÉTHODES DE TRAITEMENT DE DOULEURS NEUROPATHIQUES

(30) Priority: 08.12.2017 GB 201720505
(43) Date of publication of application: 14.10.2020
(73) Proprietor: Volution Immuno Pharmaceuticals SA, 1211 Genève 3 (CH)
(72) Inventor: CHAZOT, Paul Louis, Durham DH1 3LE (GB); NUNN, Miles Andrew, 1211, 3 Geneve (CH); OBARA, Ilona Genowefa, Newcastle upon Tyne NE1 7RU (GB)
(74) Representative: Carpmaels & Ransford LLP
(86) International application number: PCT/EP2018/084036
(87) International publication number: WO 2019/110825

(56) References cited:
- WO-A1-99/27104
- WO-A1-2009/141621
- OBARA I. ET AL: "REVISITING HISTAMINE SYSTEM FORNEUROPATHIC PAIN RELIEF: NOVEL NATURALPRODUCT STRATEGY", INFLAMM. RES., vol. 67, no. Suppl. 1, 2 June 2018 (2018-06-02), page S9, XP002789253,
- DATABASE MEDLINE [Online] US NATIONAL LIBRARY OF MEDICINE (NLM), BETHESDA, MD, US; December 2015 (2015-12), SANNA MARIA D ET AL: "Histamine H4 receptor activation alleviates neuropathic pain through differential regulation of ERK, JNK, and P38 MAPK phosphorylation.", XP002789254, Database accession no. NLM26270581
- A C ROSA ET AL: "The role of histamine in neurogenic inflammation : Histamine and neurogenic inflammation", BRITISH JOURNAL OF PHARMACOLOGY, vol. 170, no. 1, 1 September 2013 (2013-09-01), pages 38-45, XP055561549, UK ISSN: 0007-1188, DOI: 10.1111/bph.12266
- JIE YU ET AL: "A critical time window for the analgesic effect of central histamine in the partial sciatic ligation model of neuropathic pain", JOURNAL OF NEUROINFLAMMATION, vol. 13, no. 1, 1 December 2016 (2016-12-01), XP055561544, DOI: 10.1186/s12974-016-0637-0
- YU JIE ET AL: "Effects of histamine on spontaneous neuropathic pain induced by peripheral axotomy", NEUROSCIENCE BULLETIN, SHANGHAI INSTITUTES FOR BIOLOGICAL SCIENCES, CHINESE ACADEMY OF SCIENCES, HEIDELBERG, vol. 29, no. 3, 13 March 2013 (2013-03-13) , pages 261-269, XP035309165, ISSN: 1673-7067, DOI: 10.1007/S12264-013-1316-0 [retrieved on 2013-03-13]
- MARIA DOMENICA SANNA ET AL: "Histamine H 4 receptor agonist-induced relief from painful peripheral neuropathy is mediated by inhibition of spinal neuroinflammation and oxidative stress : Neuronal H 4 receptors in neuropathic pain", BRITISH JOURNAL OF PHARMACOLOGY, vol. 174, no. 1, 1 January 2017 (2017-01-01), pages 28-40, XP055561556, UK ISSN: 0007-1188, DOI: 10.1111/bph.13644
- T. CHAUMETTE ET AL: "Effects of S 38093, an antagonist/inverse agonist of histamine H3 receptors, in models of neuropathic pain in rats", EUROPEAN JOURNAL OF PAIN, vol. 22, no. 1, 6 September 2017 (2017-09-06), pages 127-141, XP055561739, GB ISSN: 1090-3801, DOI: 10.1002/ejp.1097

## Description

Methods of treating neuropathic pain are provided. In particular, the invention provides a method for treating neuropathic pain related mechanical hypersensitivity (allodynia and hyperalgesia) and peripheral neuropathic pain.

### Neuropathic pain

Neuropathic pain is pain that is caused by a lesion or disease of the somatosensory nervous system. The somatosensory system allows for the perception of touch, pressure, pain, temperature, position, movement and vibration [1]. Neuropathic pain is associated with peripheral and central sensitization (increase response of neurons) that may lead to development of allodynia (pain arising from normally non-painful stimuli such as light touching), hyperalgesia (increased sensitivity to painful stimuli), or may be associated with abnormal sensations called dysesthesia. It may be continuous and/or resemble stabbings or electric shocks. Common qualities include burning or coldness, "pins and needles" sensations, numbness and itching, excruciating pain and difficulty correctly sensing temperatures. Neuropathic pain is estimated to affect approximately 7-10% of the general population [1]. It can significantly impair quality of life as sufferers often also exhibit related problems such as disturbed sleep, depression, anxiety, loss of function and impaired cognition. Neuropathic pain is described in detail in a review by Colloca, L. et al. [1]I.

Neuropathic pain is mechanistically dissimilar to other chronic pain conditions such as inflammatory pain that occurs, for example, in rheumatoid arthritis, in which the primary cause is inflammation [1]. According to the Brain and Spine Foundation's website, "common causes of neuropathic pain include nerve pressure or nerve damage after surgery or trauma, viral infections, cancer, vascular malformations, alcoholism, neurological conditions such as multiple sclerosis and metabolic conditions such as diabetes. It may also be a side effect of certain medications e.g. chemotherapy. Occasionally no identifiable cause is found which can be distressing for the individual experiencing the pain. Chronic neuropathic pain is common and may be related to an underlying health condition such as cancer or diabetic neuropathy, or it could be related to treatments such as chemotherapy [2]. Chronic neuropathic pain may affect or follow the lower back, lower limbs, neck and upper limbs".

Neuropathic pain can be subtyped according to the location of its pathology. Peripheral neuropathic pain is generally caused by pathology of the peripheral nerves and often affects one or more distal extremity, for example, the feet, calves, hands and forearms. Central neuropathic pain, on the other hand, is caused by a lesion or disease of the spinal cord and/or brain [1].

Standard over-the-counter painkillers such as non-steroidal anti-inflammatory drugs (e.g. paracetamol, ibuprofen and aspirin) usually are not effective for neuropathic pain. A number of other treatments are available, but a large variation in response between patients to the type and dose of the pharmacological or non-pharmacological agent means it is often a "trial and error" process to determine which treatment will be best for a particular individual. Existing treatments include anti-epileptics (e.g. gabapentin and pregabalin), anti-depressants (e.g. amitriptyline, duloxetine), opioids (e.g. codeine, dihydrocodeine and morphine, oxycodone, fentanyl, buprenorphine), capsicum cream, lidocaine patch, injections/nerve blocks (usually a combination of a local anaesthetic agent, opioids and steroids), transcutaneous electric nerve stimulation (TENS), percutaneous electrical nerve stimulation (PENS) and acupuncture [2]. Pregabalin (a GABA analogue), gabapentin (unknown mechanism), duloxetine (a serotonin-noradrenaline reuptake inhibitor) and various tricyclic antidepressants are recommended as first-line treatments for peripheral and central neuropathic pain [1,3]. The aim of existing treatments is to make the pain as manageable as possible whilst minimizing any negative side effects of the treatment. All current treatments listed cause a variable range of serious central nervous system (CNS) side effects including sedation, psychological changes, headache, nausea, visual problems, restlessness, lack of attention, and nightmares. Use of opioids is effective only short term but leads to tolerance and dependence, and loses efficacy long term. New effective CNS-sparing treatments are needed with reduced side effects.

WO 99/27104 A1 describes histamine and serotonin binding molecules. WO 2009/141621 describes histamine binding proteins. Sanna et al. (2015) Pain, 156(12):2492-2504 describes histamine H4 receptor activation alleviates neuropathic pain through differential regulation of ERK, JNK, and P38 MAPK phosphorylation. Rosa et al. (2013) British Journal of Pharmacology, 170(1):38-45, describes the role of histamine in neurogenic inflammation. Yu et al. (2016) Journal of Neuroinflammation, 13:163 describes a critical time window for the analgesic effect of central histamine in the partial sciatic ligation model of neuropathic pain. Yu et al. (2013) Neuroscience Bulletin, 29:261-269 describes effects of histamine on spontaneous neuropathic pain induced by peripheral axotomy. Sanna et al. (2016) British Journal of Pharmacology, 174(1):28-40 describes histamine H4 receptor agonist-induced relief from painful peripheral neuropathy is mediated by inhibition of spinal neuroinflammation and oxidative stress. Chaumette et al. (2017) European Journal of Pain, 22(1):127-141 describes effects of S38093, an antagonist/inverse agonist of histamine H3 receptors, in models of neuropathic pain in rats.

### Summary of the invention

The invention provides an histamine binding protein (HBP) for use in a method of treating neuropathic pain.

Also provided is a purified nucleic acid molecule encoding an HBP for use in a method of treating neuropathic pain.

Also provided is a vector comprising a purified nucleic acid molecule as described herein for use in a method of treating neuropathic pain.

Also provided is a pharmaceutical composition comprising an HBP, a purified nucleic acid or a vector as described herein, in conjunction with a pharmaceutically-acceptable carrier, for use in a method of treating neuropathic pain.

### Detailed description of the invention

References herein to a method of treatment using an HBP, polynucleotide, or vector should be interpreted as references to the HBP, polynucleotide, or vector for use in the method of treatment. Similarly, references to uses of an HBP, polynucleotide, or vector in the manufacture of a medicament for treatment should be interpreted as references to the HBP, polynucleotide, or vector for use in the method of treatment.

The invention provides a histamine binding protein for use in a method of treating neuropathic pain.

The vasoactive amine histamine is known to be a mediator of inflammation and a regulator of certain physiological processes in animals, including humans. Histamine is present in the secretory granules of mast cells and basophils and is formed by decarboxylation of histidine. It is also present in ergot and plants and may be synthesised synthetically from histidine or citric acid. Histamine produces its actions by an effect on specific histamine receptors which are of four main types, H1, H2, H3 and H4, distinguished by means of selective antagonist and agonist drugs [4].

The main actions of histamine in humans are stimulation of gastric secretion, contraction of most smooth muscle, cardiac stimulation, vasodilation, increased vascular permeability, and wakefulness through CNS action. In addition to its regulatory role in immune reactions and inflammatory processes, histamine also modulates the production of many cytokines in the body (including those that regulate inflammation) and can interfere with the expression of cytokine receptors. Furthermore, histamine promotes wound healing. It is thought that the main pathophysiological roles of histamine are as a stimulant of gastric acid secretion and as a mediator of type I hypersensitivity reactions such as urticaria and hay fever. Histamine or its receptors may also be involved either directly or indirectly in autoimmune disease, e.g. arthritis, and in tumour growth [4]. Recently, a role for histamine has been implicated in the sensory nervous system and antihistamines (targeting H3 and H4 receptors) have been reported to modify neuropathic pain in animal models, although results are contradictory with both agonists and antagonists being apparently effective [5]. We believe this is due in part to the CNS action of the antihistamines used. Use of a histamine-binding protein, in particular a CNS-sparing histamine-binding protein, is a novel strategy for treating neuropathic pain, as described herein. The findings of the present invention emphasise the importance of the peripheral histamine system in the modulation of neuropathic pain, in particular in the modulation of mechanical hypersensitivity (allodynia) and peripheral neuropathies.

The present invention provides a histamine binding protein (HBP) for use in a method of treating neuropathic pain. In some embodiments, the HBP is derived from or derivable from an arthropod. In some embodiments, the histamine binding protein used in the invention is derived from or derivable from blood-feeding parasites or venom-producing animals such as venomous snakes and spiders. However, any other suitable histamine binding protein may be used. Preferably, the HBPs used in the present invention are derived from or derivable from blood-feeding ectoparasites. Most preferably, they are derived from or derivable from ticks, for example, from the *Rhipicephalus appendiculatus, Dermacentor reticulatus* or *Ornithodoros moubata* tick, more preferably from tick saliva. It is known that blood-feeding ectoparasites, such as ticks, produce numerous bioactive proteins that immunomodulate the host response to parasite feeding and thereby promote parasite blood-feeding.

The existing treatments of neuropathic pain described above are primarily small-molecule compounds, whereas the HBPs described herein are proteins. These HBPs have several benefits over small molecule inhibitors as they act through the H1, H3 and H4 histamine receptors. As a result HBPs have the dual-effect of acting as an analgesic and an anti-inflammatory, which is generally not seen with existing treatments which primarily act as analgesics.

In some embodiments, the histamine binding protein is a tick protein as described in WO 97/44451. In some embodiments, the histamine binding protein is selected from FS-HBP2, FS-HBP1, MS-HBP1 and D.RET6, or a variant thereof. These HBPs are described in detail in WO 97/44451. In some embodiments, the histamine binding protein (HBP) is FS-HBP2 and comprises or consists of the sequence presented in SEQ ID NO:2 (or amino acids 20-190 of SEQ ID NO: 2). In some embodiments, the HBP is FS-HBP1 and comprises or consists of the sequence presented in SEQ ID NO:4 (or amino acids 19-190 of SEQ ID NO: 4). In some embodiments, the HBP is MS-HBP1 and comprises or consists of the sequence presented in SEQ ID NO:6 (or amino acids 19-200 of SEQ ID NO: 6). In some embodiments, the HBP is D.RET6 and comprises or consists of the sequence presented in SEQ ID NO:8 (or amino acids 29-209 of SEQ ID NO: 8).

FS-HBP2 is well known in the prior art [6,7,8,9] and is also known as EV131. EV131 has previously been found to be effective in treating various diseases and conditions, including conjunctivitis and pruritis. However, none of these suggest a role in treating neuropathic pain. The experiment described in the examples describes the first evidence for the analgesic effect of EV131 upon neuropathic pain related mechanical hypersensitivity and its CNS-sparing action, thus emphasizing the importance of histamine binding proteins in the modulation of peripheral neuropathies.

In some embodiments, the HBP is a CNS-sparing HBP. In some embodiments, the HBP does not cross the blood-brain barrier.

### Therapeutic use

The HBPs of the invention, as described herein, are for use in a method of treating neuropathic pain. Advantageously, the invention provides a method for treating neuropathic pain in which histamine plays a role. For example, the invention provides a method for treating neuropathic pain in which the method involves preventing histamine from binding to one or more of its receptors, for example, preventing histamine from binding to any one or more of the H1, H2, H3 and H4 receptors. Preferably, histamine is prevented from binding to any of the H1, H2, H3 and H4 receptors. Preferably, histamine is prevented from binding to any of its receptors in the periphery. All four receptors are found in the CNS and peripheral nervous system (PNS).

In some embodiments, the neuropathic pain is associated with allodynia (pain arising from normally non-painful stimuli such as light touching) that is observed as hypersensitivity to mechanical stimuli. Examples of mechanical hypersensitivity include allodynia (clinical feature of many painful conditions) in e.g. neuropathies (e.g. diabetes, post chemotherapy), complex regional pain syndrome, postherpetic neuralgia, fibromyalgia, and migraine. Thus, in some embodiments, the HBP is for use in treating neuropathic pain related mechanical hypersensitivity (also referred to herein as hypersensitivity to mechanical stimuli). In some embodiments, the neuropathic pain related mechanical hypersensitivity is allodynia. In some embodiments, the neuropathic pain related mechanical hypersensitivity is hyperalgesia. In some embodiments, the neuropathic pain related mechanical hypersensitivity is allodynia and hyperalgesia. In some embodiments, the neuropathic pain is associated with hypersensitivity to thermal stimuli. In some embodiments, the neuropathic pain is not associated with hypersensitivity to thermal stimuli. In some embodiments, the HBP is for use in treating neuropathic pain related mechanical hypersensitivity without affecting neuropathic pain related thermal hypersensitivity.

In some embodiments, the treatment results in improvements in the sensations of one or more of touch (including light touch), burning or coldness, "pins and needles", numbness, itching, excruciating pain and difficulty correctly sensing temperatures. In some embodiments, the treatment results in improvements in the sensations of one or more of touch (including light touch), "pins and needles", numbness, itching and excruciating pain.

In some embodiments, the treatment eliminates the neuropathic pain. In some embodiments, the treatment reduces the symptoms of neuropathic pain (allodynia and/or hyperalgesia). In some embodiments, the treatment makes the neuropathic pain more manageable even if it does not eliminate it (i.e., improves Quality of Life (QoL) measures).

Standard tests are available in the art for assessing whether neuropathic pain has been treated using a particular agent. These will be well known to the skilled person. For example, the assessment of pain sensitivity in humans has been standardized using quantitative sensory testing (pinpricks, pressure algometer, von Frey filaments, touch, pinching, a light pressure with a finger) [10].

In some embodiments, administration of an HBP as described herein reduces the symptoms of neuropathic pain, or eliminates neuropathic pain, as assessed by sensory testing (e.g. using one or more standard tests such as those described herein, pinpricks, von Frey filaments, light touch).

Preferably, use of the HBP of the invention in a particular patient does not have any side-effects or does not have one or more of the negative side effects that are often associated with the use of other existing treatments for neuropathic pain. Such side effects include somnolence, weight gain, nausea, vomiting, abdominal pain, constipation, hypertension, sedation, dizziness, memory loss, peripheral oedema, local erythema, itching, rash, pain and anticholinergic effects. Preferably, use of the HBP of the invention does not result in any of these side effects. Significantly, no serious side effects were noted in any of the studies reported when EV131 was tested for treatment of asthma in WO 2009/141621. In some embodiments, use of the HBP of the invention does not result in any serious side effects.

In some embodiments, the neuropathic pain is peripheral. In some embodiments, the neuropathic pain is central. In some embodiments, the neuropathic pain is peripheral but not central. In some embodiments, the neuropathic pain is a mixture of both peripheral and central (e.g. Parkinson's disease).

Preferably, the neuropathic pain is peripheral. In some embodiments, the patient has peripheral neuropathy (e.g. diabetic neuropathy). In some embodiments, the patient has sensory peripheral neuropathy. In some embodiments, the peripheral neuropathic pain is caused by damaged, irritated or diseased peripheral nerves. For example, in some embodiments the patient has peripheral nerve injury-induced neuropathic pain. In some embodiments, the pain affects one or more distal extremity selected from one or more of the feet, calves, hands and forearms. In some embodiments, the sensory ganglia is affected and the pain affects one or more of the trunk, thighs and upper arms. In some embodiments, the peripheral neuropathic pain involves the small unmyelinated C fibres and/or the myelinated A fibres, namely the Aβ and Aδ fibres. The ageing global population, growing sedentary lifestyle, the increased incidence of diabetes mellitus and neurodegenerative disease, and the increasing use of chemotherapy, all affect sensory fibres (Aβ, Aδ and C fibres) and so peripheral neuropathic pain will probably become more common in the future. In some embodiments, the patient is more than 50 years old, e.g. more than 55, 60, 65, 70, 75, 80, 85 or 90 years old. In some embodiments, the patient has diabetes mellitus or diabetic (poly)neuropathy. In some embodiments, the patient is undergoing treatment with chemotherapy or has previously undergone treatment with chemotherapy. In some embodiments, the peripheral neuropathic pain is caused by injury of the sciatic nerve, for example, by chronic constriction injury of the sciatic nerve. In some embodiments, the patient has peripheral nerve problems such as neuropathy caused by spinal stenosis. In some embodiments, the patient has a peripheral disorder selected from one or more of trigeminal neuralgia, postherpetic neuralgia, peripheral nerve injury pain, post-amputation pain, painful polyneuropathy and painful radiculopathy. In some embodiments, the patient has a peripheral disorder associated with spinal cord injury and/or failed hip or back surgery.

In some embodiments, the peripheral neuropathic pain disorder has a generalized distribution. In some embodiments, the distribution of the pain is symmetrical. In some embodiments, the patient has a generalized peripheral neuropathy associated with a disease, condition or treatment selected from diabetes mellitus, pre-diabetes, metabolic dysfunction, an infectious disease (e.g. HIV infection or leprosy), chemotherapy, an immune disorder (e.g. Guillain-Barré syndrome), an inflammatory disorder, or an inherited neuropathy and channelopathy (e.g. inherited erythromelalgia).

In some embodiments, the peripheral neuropathic pain disorder has a focal distribution. In some embodiments, one or more peripheral nerves or nerve roots are affected. In some embodiments, the patient has a focal peripheral disorder selected from postherpetic neuralgia, post-traumatic neuropathy, postsurgical neuropathy, cervical and lumbar polyradiculopathy, pain associated with HIV infection, leprosy, diabetes mellitus, complex regional pain syndrome type 2 and trigeminal neuralgia.

In some embodiments, the peripheral neuropathic pain is experienced within the facial or intra-oral trigeminal nerve territory. In some embodiments, the peripheral neuropathic pain has a unilateral distribution in one or more spinal dermatome or the trigeminal nerve territory (usually the ophthalmic division). In some embodiments, the peripheral neuropathic pain is experienced in the innervation territory of the injured nerve, typically distal to a site of surgery, trauma or compression. In some embodiments, the peripheral neuropathic pain is experienced in a missing body part or in a residual limb. In some embodiments, the peripheral neuropathic pain is experienced in the innervation territory of the affected nerve root.

Central neuropathic pain is caused by a lesion or disease of the spinal cord and/or brain [1]. In some embodiments, the patient has central neuropathic pain caused by a lesion or disease of the spinal cord. In some embodiments, the patient has central neuropathic pain caused by a lesion or disease of the brain. In some embodiments, the patient has central neuropathic pain caused by a lesion or disease of the spinal cord and brain. In some embodiments, the patient has cerebrovascular disease affecting the central somatosensory pathways (post-stroke pain) or neurodegenerative disease (e.g. Parkinson disease). In some embodiments, the patient has a spinal cord lesion or disease, for example, selected from spinal cord injury, syringomyelia and demyelinating diseases, such as multiple sclerosis, transverse myelitis and neuromyelitis optica. These cause central neuropathic pain.

In some embodiments, the neuropathic pain is chronic. In some embodiments, the neuropathic pain is acute.

In some embodiments, the patient has neuropathic pain that is caused by one or more of the following: nerve pressure or nerve damage after surgery or trauma, a viral infection, cancer, a vascular malformation, alcoholism, a neurological condition such as multiple sclerosis, a metabolic condition such as diabetes, a side effect of medication or chemotherapy.

### Variants and fragments of the HBPs

The invention also comprises variants of the HBPs described herein and variants of other known or naturally-occurring HBPs for use in a method of treating neuropathic pain. Variants include natural biological variants (e.g. allelic variants or geographical variations within the species from which the HBPs are derived). Variants also include non-naturally occurring variants, such as synthetic variants. The present invention also includes proteins belonging to the same protein family as the HBPs described herein. Fragments of the histamine binding proteins, fragments of the variants and fragments of the proteins belonging to the same family as the HBPs described herein are also included in the invention.

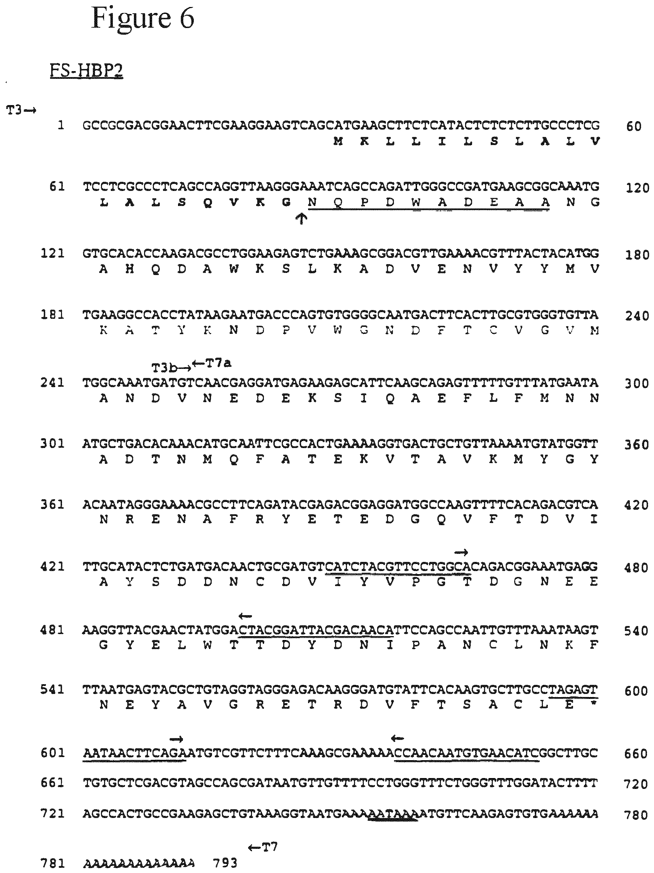

In some embodiments, a variant has (*e.g*. comprises or consists of) a protein sequence having at least 70% sequence identity (e.g. at least 75%, 80%, 85%, 90%, 95%, 96%, 97%, 98%, 99% sequence identity) to a known HBP sequence, e.g. to an HBP sequence as described herein. For example, in some embodiments, the HBP used in the invention is a variant that has (e.g. comprises or consists of) a protein sequence having at least 70% sequence identity (e.g. at least 75%, 80%, 85%, 90%, 95%, 96%, 97%, 98%, 99% sequence identity) to SEQ ID NO:2. Other examples of variants include variants of mature proteins (i.e. SEQ ID NOs 2, 4, 6, or 8 that lack the signal sequences (e.g. as shown in Figures 6-9 and referred to in WO 97/44451, e.g. amino acids 20-190, 19-190, 19-200 and 29-209 of SEQ ID NOs 2, 4, 6, and 8, respectively). Sequence identity between polynucleotide sequences is preferably determined by pairwise alignment algorithm using the Needleman-Wunsch global alignment algorithm [11], using default parameters (e.g. with Gap opening penalty = 10.0, and with Gap extension penalty = 0.5, using the EDNAFULL scoring matrix). This algorithm is conveniently implemented in the needle tool in the EMBOSS package [12]. In this way, sequence identity is calculated over the entire length of the reference polypeptide sequence. The needle tool in the EMBOSS package can also be used to determine sequence identity between polypeptide sequences.

In some embodiments, a variant comprises a protein sequence as described herein (e.g. SEQ ID NO:2, 4, 6, 8, or amino acids 20-190, 19-190, 19-200 and 29-209 of SEQ ID NOs 2, 4, 6, and 8 respectively) or comprises the protein sequence of another naturally occurring HBP protein sequence that contains single or multiple amino-acid substitution(s), addition(s), insertion(s) and/or deletion(s) from the given protein sequence and/or substitutions of chemically-modified amino acids. In some embodiments, the variant comprises no more than 1, 2, 3, 4, 5, 6, 7, 8, 9, 10, 12, 15, 18, 19 or 20 such substitutions, additions, insertions or deletions.

Preferably, the variant retains the biological function of binding to histamine. The ability of the HBP to bind to histamine may be measured directly by detection of HBP-histamine complexes. In some embodiments, the variant prevents histamine from binding to one or more of the H1, H2, H3 and H4 receptors, for example, to any of the H1, H2, H3 and H4 receptors. In some embodiments, the variant prevents histamine from binding to any of its receptors in the periphery. The ability of the HBP to prevent histamine from binding to one or more of its receptors may be measured indirectly, for example by an assay which measures a change in a cell caused by the reduction in histamine levels due to HBP activity. For example, it may be measured by assessing inhibition of histamine-induced IL-6 production by HUVEC cells. Preferably, the HBP can inhibit such IL-6 production by at least 80%. The variant is preferably able to reduce neuropathic pain related mechanical hypersensitivity in an adult male C57BL/6J (B6) mouse where the pain has been induced by chronic constriction injury of the sciatic nerve, for example, where the mouse is administered the variant at a dose of 10 mg/kg i.p. (intraperitoneal) using a regimen of 4 injections every 24 hours for 4 days. Preferably the variant can reduce neuropathic pain related mechanical hypersensitivity in the adult male C57BL/6J (B6) mouse to the same or greater extent than EV131 can e.g. when administered under the same conditions and at the same dosages.

In some embodiments, the fragment is an active fragment of an HBP, for example an active fragment of EV131 (e.g. an active fragment of the protein with the sequence listed in SEQ ID NO:2) or an active fragment of a variant of a known HBP sequence (e.g. an active fragment of a variant of SEQ ID NO:2), wherein "active fragment" denotes a truncated protein that retains the biological function of binding to histamine. Examples of methods for determining the ability to bind to histamine are described above. In some embodiments, the fragment prevents histamine from binding to one or more of the H1, H2, H3 and H4 receptors, for example, to any of the H1, H2, H3 and H4 receptors. In some embodiments, the fragment prevents histamine from binding to any of its receptors in the periphery. Examples of methods for determining the ability to prevent histamine from binding to its receptors are described above. The fragment is preferably able to reduce neuropathic pain related mechanical hypersensitivity in an adult male C57BL/6J (B6) mouse where the pain has been induced by chronic constriction injury of the sciatic nerve, for example, where the mouse is administered the variant at a dose of 10 mg/kg i.p. using a regimen of 4 injections every 24 hours for 4 days. Preferably the fragment can reduce neuropathic pain related mechanical hypersensitivity in the adult male C57BL/6J (B6) mouse to the same extent that EV131 can.

In some embodiments, the fragment is at least 80 amino acids in length, e.g. at least 100, 120, 140, 150, 160, 170, 180, 185, or 188 amino acids in length. In some embodiments, the fragment is truncated by no more than 1, 2, 3, 4, 5, 10, 15, 19, 20 or 25 amino acids from the N- and/or C-terminal of the full length HBP sequence. An active fragment of an HBP does not contain the full length HBP sequence. As discussed elsewhere, specific examples of active fragments include fragments of SEQ ID NOs 2, 4, 6, and 8 (or variants thereof) that lack the amino acids 1-19, 1-18, 1-18 and 1-28 of SEQ ID NOs 2, 4, 6, and 8, respectively.

In some embodiments, the fragment comprises the sequence presented in SEQ ID NO:10. This sequence corresponds to the sequence of FS-HBP2 having SEQ ID NO:2. However, the protein lacks the first 19 amino acids of the FS-HBP2 sequence. The sequence therefore commences at position 20 of the FS-HBP2 sequence. However, additionally, a methionine residue has been appended to the sequence at the N terminus. Furthermore, at position 146 in the amino acid sequence of the protein of the invention, there is a Leucine residue, replacing the Proline residue that occupies position 164 in the amino acid sequence of the FS-HBP2 protein. This protein is described in WO 2009/141621. SEQ ID NO: 10 is an example of an HBP that lacks the signal sequence and thus can be described as a "mature protein". Such "mature proteins" thus can be described as fragments of HBPs. A further example of such a mature protein is SEQ ID NO: 11.

The HBP used in the invention may form part of a fusion protein. For example, it is often advantageous to include one or more additional amino acid sequences which may contain secretory or leader sequences, pro-sequences, sequences which aid in purification, or sequences that confer higher protein stability, for example during recombinant production, or that renders the polypeptide detectable by imaging technology. For instance, a derivative may include an additional protein or polypeptide fused to the HBP at its amino- or carboxy-terminus or added internally to the HBP. The purpose of the additional polypeptide may be to lend additional properties to the HBP as desired. Examples of potential fusion partners include β-galactosidase, glutathione-S-transferase, luciferase, polyhistidine tags, T7 polymerase fragments and secretion signal peptides. Other examples include extracellular domains of membrane-bound proteins, immunoglobulin constant regions (Fc regions), multimerization domains, domains of extracellular proteins, signal sequences, export sequences, and sequences allowing purification by affinity chromatography or sequence allowing imaging, for example fluorescent polypeptides. Other examples will be clear to those of skill in the art. For instance, an HBP used in the invention may further comprise a histidine tag, preferably located at the C-terminal of the polypeptide, generally comprising between 1-10 histidine residues, particularly 6 histidine residues. In some embodiments, the fusion protein comprises an HBP which is a variant or a fragment as described herein. Preferably the fragment is an active fragment.

The HBP used in the invention binds to histamine with high affinity. Preferably, the HBP binds to histamine with a dissociation constant of less than 10⁻⁷M, more preferably, less than 10⁻⁸M, less than 10⁻⁹M or less than 10⁻¹⁰M. For example, the HBP may bind to histamine with a dissociation constant of less than 10⁻⁷M but greater than 10⁻¹²M, more preferably, less than 10⁻⁸M but greater than 10⁻¹¹M, or less than 10⁻⁹M but greater than 10⁻¹⁰M.

In some embodiments, the HBP specifically binds to histamine with a dissociation constant of less than 10⁻⁷M and belongs to the same protein family as MS-HBP1, FS-HBP1, FS-HBP2 and D.RET6. In some embodiments, a protein is considered to belong to this family if 70% or more of the amino acids that are completely conserved as identical residues in the alignment of the four HBPs alone, are still completely conserved as identical residues if the protein is included in the alignment, the alignments being obtained using GCG's pileup command (Program manual for the Wisconsin package, 1994; gap creating penalty = 2.50; gap extension penalty = 0.05). Also included as a member of this HBP family are those proteins from haematophagous arthropods that bind histamine with an affinity characterised by a dissociation constant less than 10⁻⁷M and contain the sequence motifs D/E A W K/R and Y/C E/D L/I W.

In some embodiments, the HBP is selected from:
(a) any histamine binding protein that binds specifically to histamine with a dissociation constant of less than 10⁻⁷ M and which belongs to the same protein family as the proteins MS-HBP1, FS-HBP1 and FS-HBP-2 disclosed in WO97/44451, wherein a protein is considered to belong to this protein family if the primary, mature monomer sequence of the protein has no more than 260 amino acids and at least 30 of the amino acids in the protein's complete sequence are conserved as identical residues in an alignment of that protein and the proteins MS-HBP1, FS-HBP1 and FS-HBP-2, the alignment preferably having been obtained using GCG's pileup command (Program Manual for the Wisconsin Package, 1994; gap creating penalty = 3; gap extension penalty = 1, scoring matrix Blosum62.cmp, pileup carried out using the endweight option);
(b) a protein from a haematophagous arthropod that binds specifically to histamine with a dissociation constant less than 10⁻⁷ M and which contains the sequence motifs D/E A W K/R (preferably DAWK, more preferably QDAWK) and Y/C E/D L/I/F W (preferably Y/C ELW);
(c) a natural biological variant, such as an allelic variant or a geographical variants, of a protein as defined in (a) or (b) above;
(d) a functional equivalent of a protein as defined in (a), (b) or (c) above that contains single or multiple amino-acid substitution(s), addition(s), insertion(s) and/or deletion(s) from the wild type protein sequence and/or substitutions of chemically-modified amino acids that do not affect the biological function of binding to its respective vasoactive amine;
(e) an active fragment of a protein as defined in (a), (b), (c) or (d) above, wherein "active fragment" denotes a truncated protein that retains the biological function of binding to histamine (e.g. a protein lacking its signal sequence as defined elsewhere herein); and
(f) a fusion protein comprising a protein as defined in (a), (b), (c), (d) or (e) above fused to a peptide or other protein, such as a label, which may be, for instance, bioactive, radioactive, enzymatic or fluorescent, or an antibody.

In some embodiments, a protein is in the same family as the MS-HBP1, FS-HBP1 and FS-HBP-2 proteins disclosed in WO97/44451 if it contains more than 40, more preferably more than 50, more preferably more than 60 residues, most preferably 70 residues or more which are identical as defined in a) above when aligned with the proteins shown in Table 1.

The HBP used in the invention also binds *specifically* to histamine. The term "specifically" means that the HBP has substantially greater affinity for histamine than for other compounds. By "substantially greater affinity" we mean that there is a measurable increase in the affinity for an HBP used in the invention for histamine as compared with its affinity for other compounds. Preferably, this measurable increase in affinity is at least 10-fold, 100-fold, 10³-fold, 10⁴-fold, 10⁵-fold, 10⁶-fold or greater for histamine than for other compounds. Methods for measuring specificity will be known to those of skill in the art and include competition assays and the like.

The HBPs described herein have been found to be stable. Preferably, the HBP used in the invention is stable. For example, the protein is preferably stable at room temperature (approximately 19°C to 25°C or approximately 20°C). The half-life of the protein is preferably over one hour, preferably over 5 hours, preferably over 10 hours, preferably over 24 hours, more preferably over 48 hours or more, at room temperature. The HBP is preferably stable during storage at 4°C or at a room temperature of 25°C for at least 52 weeks. Preferably, the half-life of the protein is over one week, preferably over two weeks, preferably over 4 weeks, preferably over 12 weeks, preferably over 26 weeks, preferably over 52 weeks or more at room temperature or at a storage temperature (approximately 4°C). This facilitates working with the HBP, and makes it easier, for example, for it to be manipulated and administered as a drug to a patient.

Stability of the HBP can be measured by assessing whether it retains molecular integrity over time, for example, whether its molecular weight is altered over time as a result of either degradation or aggregation. This can be assessed by standard methods known in the art, such as SDS-PAGE. Stability can also be measured by assessing the activity of the protein, since a stable protein preparation will have retained substantially of all of its histamine binding affinity. Ability of the HBP to retain histamine binding activity may be measured directly by detection of HBP-histamine complexes. Alternatively, the ability of the HBP to retain histamine binding activity may be measured indirectly, for example by an assay which measures a change in a cell caused by the reduction in histamine levels due to HBP activity. For example, removal of histamine by HBP decreases histamine- dependent IL-6 production in TNFα activated human umbilical vein endothelial cell (HUVEC) monolayers. The stability of the HBP may therefore be assessed by determining the effect of HBP on IL-6 release by such cells. Preferably, the HBP of the invention inhibits histamine-induced IL-6 production by HUVEC cells by at least 80%, preferably at least 90%, preferably at least 95%, after storage at room temperature or at a storage temperature (approximately 4°C) for at least 52 weeks.

The stability of the protein can also be estimated from its sequence, for example, using a bioinformatics tool (ProtParam (ExPASy, Switzerland)). As assessed in this manner, the estimated half-life of the protein is preferably between 20 and 40 hours, more preferably approximately 30 hours in a representative mammalian system (mammalian reticulocytes, *in vitro);* the estimated half-life of the protein is preferably greater than 10 hours, more preferably greater than 20 hours in yeast *in vivo,* the estimated half-life of the protein is preferably greater than 5 hours, and more preferably greater than 10 hours in *Escherichia coli, in vivo.*

The HBP used in the invention preferably has a half-life in rats of at least 7 hours. Preferably, the HBP has a half-life in a mammal *in vivo,* preferably a human, of greater than 2 hours, preferably greater than 5 hours, preferably greater than 6 hours, preferably greater than 7 hours. Half-life *in vivo* may be increased by conjugation or fusion of the HBP to molecules known in the art for this purpose, e.g. polyethylene glycol.

The HBPs used in the present invention can be prepared using known techniques of molecular biology or protein chemistry (for example, chemical peptide synthesis). The HBPs are preferably prepared using the known techniques of genetic engineering as described, for example, by Sambrook et al., Molecular Cloning; A Laboratory Manual, Second Edition (1989), Volumes I and II (D.N Glover ed. 1985); B. Perbal, A Practical Guide to Molecular Cloning (1984); Gene Transfer Vectors for Mammalian Cells (J.H. Miller and M.P. Calos eds. 1987, Cold Spring Harbor Laboratory); Scopes, (1987) Protein Purification: Principles and Practice, Second Edition (Springer Verlag, N.Y.). For example, HBPs used in the present invention may be prepared in recombinant form by expression in a host cell. A further aspect of the invention thus provides a method for preparing a HBP of the invention which comprises culturing a host cell containing a nucleic acid molecule encoding an HBP for use in the invention under conditions whereby said protein is expressed and recovering said protein thus produced. Such expression methods are well known to those of skill in the art and many are described in detail by Sambrook et al., 1989. A suitable expression vector can be chosen for the host of choice. The vector may contain a recombinant DNA molecule encoding a HBP operatively linked to an expression control sequence that is recognized by the host transcription machinery. Suitable hosts include commonly used prokaryotic species, such as *E. coli,* or eukaryotic yeasts that can be made to express high levels of recombinant proteins and that can easily be grown in large quantities. Cell lines grown *in vitro* are also suitable, particularly when using virus-driven expression systems such as the *Baculovirus* expression system which involves the use of insect cells as hosts.

HBPs may also be expressed *in vivo,* for example in insect larvae or in mammalian tissues. Preferably, HBP protein is expressed in E. coli; for example, strain BLR(DE3) is suitable. For example, the protein may be expressed from a pET24a-based plasmid (Novagen), although equivalent systems are equally appropriate, as the skilled reader will be aware.

The invention also provides a purified nucleic acid molecule which encodes an HBP as described herein for use in a method of treating neuropathic pain. Such molecules include DNA, cDNA and RNA, as well as synthetic nucleic acid species. The term "purified nucleic acid molecule" preferably refers to a nucleic acid molecule for use in the invention that (1) has been separated from at least about 50 percent of proteins, lipids, carbohydrates, or other materials with which it is naturally found when total nucleic acid is isolated from the source cells; (2) is not linked to all or a portion of a polynucleotide to which the "purified nucleic acid molecule" is linked in nature; (3) is operably linked to a polynucleotide which it is not linked to in nature; or (4) does not occur in nature as part of a larger polynucleotide sequence. Preferably, the purified nucleic acid molecule used in the present invention is substantially free from any other contaminating nucleic acid molecule(s) or other contaminants that are found in its natural environment that would interfere with its use in protein production or its therapeutic, diagnostic, prophylactic or research use. Preferably, the "purified nucleic acid molecule" consists of cDNA only. cDNAs encoding the FS-HBP2, FS-HBP1, MS-HBP1 and D.RET6 HBPs are disclosed herein by way of example in SEQ ID NOs: 1, 3, 5 and 7, respectively. A cDNA encoding the active fragment of FS-HBP2 having the amino acid sequence of SEQ ID NO:10 and which lacks the 19 N-terminal amino acids of FS-HBP2, which is described above, is provided in SEQ ID NO:9. cDNAs encoding active fragments, that may, for example, be mature proteins, can also be used, e.g. the purified nucleic acid molecule may comprise of consist of nucleotides 87-599 of SEQ ID NO: 1, nucleotides 66-581 of SEQ ID NO: 3, nucleotides 72-612 of SEQ ID NO: 5, or nucleotides 85-627 of SEQ ID NO: 7. In some embodiments, the purified nucleic acid molecule comprises or consists of the nucleic acid sequence as recited in any one of SEQ ID NOs:1, 3, 5, 7 or 9, more preferably which comprises or consists of the nucleic acid sequence as recited in any one of SEQ ID Nos: 1, 3, 5 or 7, or is a redundant equivalent or fragment of any one of these sequences. Preferably, the purified nucleic acid molecule comprises or consists of the nucleic acid sequence as recited in SEQ ID NO:1 (encoding EV131) or is a redundant equivalent or fragment of this sequence.

In a further embodiment, the invention provides a purified nucleic acid molecule which hybridizes under high stringency conditions with a purified nucleic acid molecule as described herein, for use in a method of treating neuropathic pain. High stringency hybridisation conditions are defined as overnight incubation at 42°C in a solution comprising 50% formamide, 5X SSC (150 mM NaCl, 15 mM trisodium citrate), 50 mM sodium phosphate (pH7.6), 5x Denhardts solution, 10% dextran sulphate, and 20 microgram/ml denatured, sheared salmon sperm DNA, followed by washing the filters in 0.1X SSC at approximately 65°C. In a further embodiment, the purified nucleic acid molecule is substantially homologous with the said sequence. By 'substantially homologous' is meant sequences displaying at least 60% sequence homology, for example, at least 60%, 65%, 70%, 75%, 80%, 85%, 90%, 95%, 96%, 97%, 98% or 99% sequence homology.

In a still further aspect, the invention provides a vector, such as an expression vector, that contains a purified nucleic acid molecule as described above, for use in a method of treating neuropathic pain. Additionally, it may be convenient to cause the recombinant protein to be secreted from certain hosts. Accordingly, further components of such vectors may include nucleic acid sequences encoding secretion signalling and processing sequences. The invention also provides a host cell transformed with such a vector for use in a method of treating neuropathic pain.

Nucleic acid molecules according to the present invention may also be used to create transgenic animals. This may be done locally by modification of somatic cells or by germ line therapy to incorporate heritable modifications. The invention therefore also includes transformed or transfected prokaryotic or eukaryotic host cells or transgenic organisms containing a purified nucleic acid molecule according to the invention as defined above.

In a further aspect, the invention provides a pharmaceutical composition comprising an HBP, a nucleic acid molecule, a vector, or a host cell according to the aspects of the invention described above, in conjunction with a pharmaceutically-acceptable carrier for use in a method of treating neuropathic pain. Preferably, such pharmaceutical compositions comprise HBPs according to the invention, optionally including an inert carrier or carriers. The HBP may constitute the sole active component of the composition or can form part of a therapeutic package, such as a component of a pill, cream, aerosol, powder, oil or aqueous composition. In the case of an aqueous composition, this can of course be lyophilised for distribution, and the lyophilised material can eventually be reconstituted with an aqueous carrier for administration to patients. Thus any process for the preparation of a composition according to the invention may further comprise the steps of lyophilising the composition and then, optionally, reconstituting the composition with an aqueous medium.

In one embodiment, the HBP of the invention is formulated in a formulation buffer, 12.6 mM Sodium Phosphate, 124 mM Sodium Chloride, pH 7.2. The protein is then serially diluted from this stock in PBS.

In another embodiment, the HBP of the invention may be formulated as a cream, preferably a water-based cream.

In another embodiment, the HBP of the invention may be an aerosol, preferably comprising dry powder with lactose, with HFA (hydrofluoroalkane) as propellant.

Once formulated, the compositions of this aspect of the invention can be administered directly to the subject. The subjects to be treated can be animals; in particular, human subjects can be treated.

The invention also provides a method for treating a patient, comprising administering a pharmaceutical composition of the invention to the patient. The patient is preferably a human, and may be a child (e.g. a toddler or infant), a teenager or an adult, but will generally be an adult. The invention also provides HBP compositions of the invention for use as a medicament. The invention also provides the use of HBPs and other compositions of the invention in the manufacture of a medicament for treating a patient. These uses, methods and medicaments are for the treatment of neuropathic pain.

Compositions according to the invention should be administered directly to a patient in a therapeutically effective amount. The term "therapeutically effective amount" as used herein refers to an amount of HBP needed to treat, ameliorate, or prevent the targeted disease or condition, or to exhibit a detectable therapeutic or preventative effect. For any compound, the therapeutically effective dose can be estimated initially either in cell culture assays, for example, of neoplastic cells, or in animal models, usually mice, rabbits, dogs, or pigs. The animal model may also be used to determine the appropriate concentration range and route of administration. Such information can then be used to determine useful doses and routes for administration in humans.

Direct delivery may be accomplished by parenteral injection (e.g. intravenously, subcutaneously, intraperitoneally, intramuscularly, or to the interstitial space of a tissue), or by rectal, oral, vaginal, topical, transdermal, intranasal, ocular, aural, pulmonary or other mucosal administration. The precise mode of administration will depend on the location of the neuropathic pain to be treated and/or the location of the pathology causing the neuropathic pain. Preferred administrations include injection, intravenous administration, intraperitoneal administration, topical administration, subcutaneous administration and inhalation (particularly subcutaneous or topical, e.g. subcutaneous). In some embodiments, the HBP is administered by intraperitoneal injection or intraplantar injection.

HBP dosing is usually scaled to a patient's body size, measured either by body weight (kg) or by body surface area (BSA; measured in m², measured, or estimated by a combination of a patient's height and weight). Although there is no exact conversion between weight and BSA dosing, there is a good approximation: for a person of average weight and height (50th percentile for each), 25000 IU/kg = 1 MIU/m².

Treatment can be a single dose schedule or a multiple dose schedule. Any suitably effective dosage may be used. The precise effective amount for a human subject will depend upon the severity of the disease state, general health of the subject, age, weight, and gender of the subject, diet, time and frequency of administration, the particular HBP being used, drug combination(s), reaction sensitivities, and tolerance/response to therapy. This amount can be determined by routine experimentation and is within the judgement of the clinician. In some embodiments, the effective dose of an HBP referred to herein is more than 3 mg/kg, e.g. more than 5, 6, 7, 8, 9, 10, 20, 30 or 40mg mg/kg. In some embodiments, the effective dose of an HBP referred to herein is 4 to 40 mg/kg, 4 to 30 mg/kg, 4 to 20 mg/kg, 5 to 15 mg/kg, 8 to 12 mg/kg, 9 to 11 mg/kg or about 10 mg/kg. In some embodiments, one to six doses (e.g. 1, 2, 3, 4, 5 or 6), or up to 1, 2, 3, 4, 5 or 6, are given every 24 hours. For example, in some embodiments, four doses are given every 24 hours. The treatment may be administered for a period of days, weeks, months or years. Preferably, one dose is given every 24 hours.

It has been shown that lower doses can achieve effective treatment of neuropathic pain when administered locally, *i.e.* near to the site of pain, *e.g.* within 2, 5, 10 or 20 cm of the site of pain. By way of example, administration near to the site of pain is local administration, e.g. through subcutaneous administration or topical administration. For subcutaneous administration, dosages of the HBP can be, for example, 2-10 mg/kg, 4-8 mg/kg or about 6 mg/kg, *e.g*. at the same frequency as described above.

By way of example in certain embodiments the HBP consists of or comprises the amino acid sequence which is amino acids 20 to 190 of SEQ ID NO:2, or is SEQ ID NO:11, or is a sequence that has at least 70, 80, 90, 95, 98, 99% identity to amino acids 20 to 190 of SEQ ID NO:2, or to SEQ ID NO:11 and/or the neuropathic pain is peripheral neuropathic pain and/or the method of administration is subcutaneous administration. Preferably the neuropathic pain is peripheral neuropathic pain and the method of administration is subcutaneous administration. HBPs of the invention can be used as the active ingredient of pharmaceuticals. Such pharmaceuticals can be used on their own to treat patients, or can be used in conjunction with other active ingredients, for example, with other pharmaceuticals or non-pharmaceuticals used to treat neuropathic pain. Typically, the HBPs will not be mixed with any other active ingredient before administration; rather, the HBP and other active ingredient(s) will be administered as separate independent medicines in a combined protocol. Thus the invention provides (a) HBPs of the invention, and (b) a second pharmaceutical agent, for simultaneous separate or sequential administration. The invention also provides a pharmaceutical preparation or system, comprising (a) a first pharmaceutical agent, which comprises HBPs of the invention; and (b) a second pharmaceutical agent, wherein said first and second agents are either in admixture or are separate compositions e.g. for simultaneous separate or sequential administration. The invention also provides a kit comprising (a) a first pharmaceutical agent, which comprises HBPs of the invention; and (b) a second pharmaceutical agent. Examples of the second pharmaceutical agent include histamine blocking agents (e.g. CNS-sparing histamine receptor antagonist agents), and H1, H2, H3 and/or H4 receptor antagonists. Use of one or more additional types of histamine modulators may further enhance the effect of modulating histamine in neuropathic pain. Further examples of second pharmaceutical agents for neuropathic pain include agents currently known to treat neuropathic pain, for example, anti-epileptics (e.g. gabapentin and pregabalin), anti-depressants (e.g. amitriptyline, duloxetine), opioids (e.g. codeine, dihydrocodeine and morphine, oxycodone, fentanyl, buprenorphine), capsicum cream, lidocaine patch and injections/nerve blocks (usually a combination of a local anaesthetic agent, opioids and steroids). The HBP of the invention may alternatively be administered in conjunction with an alternative therapy such as transcutaneous electric nerve stimulation (TENS), percutaneous electrical nerve stimulation (PENS) or acupuncture [2]. In some embodiments, the HBP of the invention is administered in conjunction with a LTB4-inhibitor protein (e.g. esculetin) [13].

The HBPs of the invention can also be administered in combination with pharmaceutical agents known to treat Parkinson's disease or related disorders. Such agents, for example, include dopamine agonists, such as pramipexole (Mirapex), ropinirole (Requip), rotigotine (given as a patch, Neupro), and apomorphine (Apokyn). Further agents known to treat Parkinson's disease or a related disorder include, for example: levodopa; monoamine oxidase B (MAO-B) inhibitors, such as selegiline (E Idepryl, Zelapar), safinimide (Xadigo) and rasagiline (Azilect); catechol O-methyltransferase (COMT) inhibitors, such as Entacapone (Comtan) and tolcapone (Tasmar); anticholinergic medications, such as benztropine (Cogentin) or trihexyphenidyl; and amantadine.

The invention also provides the use of (a) HBPs of the invention and (b) a second pharmaceutical agent, in the manufacture of a combination medicament.

The invention also provides the use of HBPs of the invention in the manufacture of a medicament, wherein the medicament is co-administered with a second pharmaceutical agent. Similarly, the invention provides the use of a second pharmaceutical agent in the manufacture of a medicament, wherein the medicament is co-administered with HBPs micro aggregates of the invention. In some embodiments, the two agents are administered within 4 hours of each other.

The invention also provides the use of HBPs of the invention in the manufacture of a medicament, wherein the medicament is for administration to a patient who has been pre-treated with a second pharmaceutical agent. Similarly, the invention provides the use of a second pharmaceutical agent in the manufacture of a medicament, wherein the medicament is for administration to a patient who has been pre-treated with HBPs of the invention. The pre-treatment may be recent (e.g. within the 24 hours preceding administration of said medicament), intermediate (e.g. more than 24 hours previous, but no longer than 4 weeks), more distant (e.g. at least 4 weeks previous), or very distant (e.g. at least 6 months previous), with these time periods referring to the most recent pre-treatment dose. The patient may be refractory to treatment by the pharmaceutical agent that was administered in the pre-treatment.

Gene therapy may be employed to effect the endogenous production of a histamine binding protein by specific cells in a patient. Gene therapy can either occur *in vivo* or ex *vivo. Ex vivo* gene therapy requires the isolation and purification of patient cells, the introduction of the therapeutic gene and introduction of the genetically altered cells back into the patient. In contrast, *in vivo* gene therapy does not require isolation and purification of a patient's cells. The therapeutic gene is typically "packaged" for administration to a patient. Gene delivery vehicles may be non-viral, such as liposomes, or replication-deficient viruses, such as adenovirus [14] or adeno-associated virus (AAV) vectors [15,16]. For example, a nucleic acid molecule encoding a histamine binding protein may be engineered for expression in a replication-defective retroviral vector. This expression construct may then be isolated and introduced into a packaging cell transduced with a retroviral plasmid vector containing RNA encoding the polypeptide, such that the packaging cell now produces infectious viral particles containing the gene of interest. These producer cells may be administered to a subject for engineering cells *in vivo* and expression of the polypeptide *in vivo* [17].

Another approach is the administration of "naked DNA" in which the therapeutic histamine binding compound is directly injected into the bloodstream or muscle tissue.

In embodiments which describe administering an HBP of the invention, it is also envisaged that a purified nucleic acid molecule encoding the HBP, a vector comprising the purified nucleic acid molecule or a pharmaceutical composition comprising the HBP, purified nucleic acid molecule or vector may be administered in place of the HBP.

The term "comprising" as used herein also encompasses embodiments which consist of only the recited elements as well as embodiments which comprise the recited elements.

Various aspects and embodiments of the present invention will now be described in more detail, with particular reference to the HBP EV131. It will be appreciated that modification of detail may be made without departing from the scope of the invention.

### BRIEF DESCRIPTION OF THE FIGURES

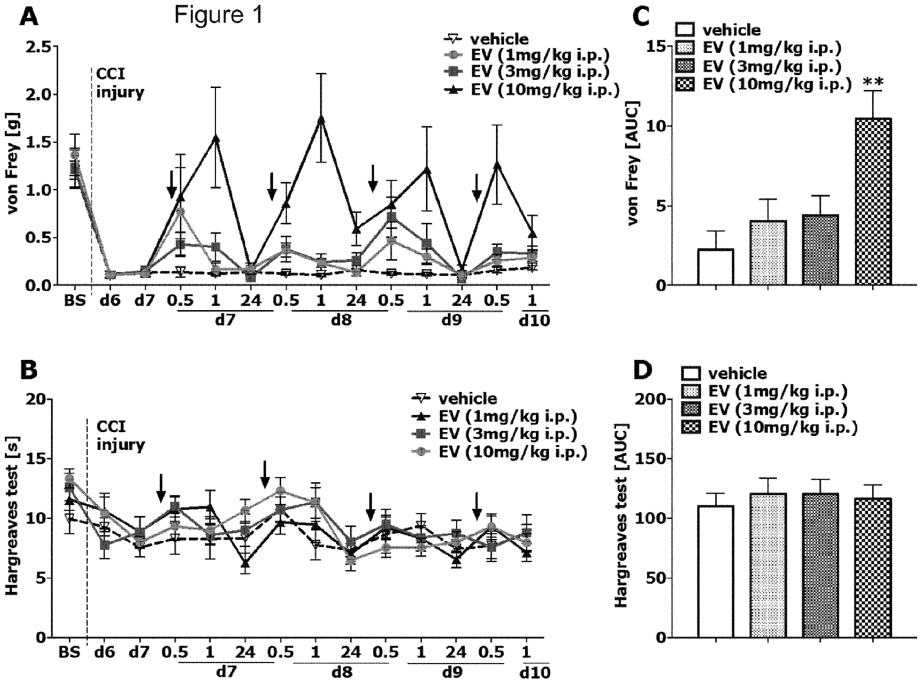

**Figure 1****:** Chronic i.p. administration of EV131 attenuated mechanical hypersensitivity but did not attenuate thermal hypersensitivity in the chronic constriction injury (CCI) model in mice. (**A-D**) Effects of systemic i.p. injection of EV131 (1, 3 or 10 mg/kg) or vehicle in CCI mice on (**A**) the mechanical withdrawal threshold measured with von Frey filaments and (**B**) the thermal withdrawal threshold measured with Hargreaves test. The measurements were assessed before injury as basal pain threshold (BS) and then 6 days following the injury. The effect of EV131 was assessed 0.5 h, 1 h and 24 h after each of four consecutive i.p. injections. The arrow indicates each of four i.p. injections of EV131. Data are presented as means ± S.E.M, n = 6 in each group. (**A**) F(39,280)=2.34 P<0.0001 vs. vehicle control animals (two-way ANOVA, followed by Bonferroni's comparison post-hoc test). (**B**) F(39,266)=1.03 P=0.43 vs. vehicle control animals (two-way ANOVA, followed by Bonferroni's comparison post-hoc test). (**C**) The area under the curve (AUC) summarizing measurements in A. F(3,20)=6.51 P<0.003 vs. vehicle control animals (one-way ANOVA, followed by Bonferroni's comparison post-hoc test) (**D**) The area under the curve (AUC) summarizing measurements in B. F(3,20)=0.16 P=0.92 vs. vehicle control animals (one-way ANOVA, followed by Bonferroni's comparison post-hoc test).

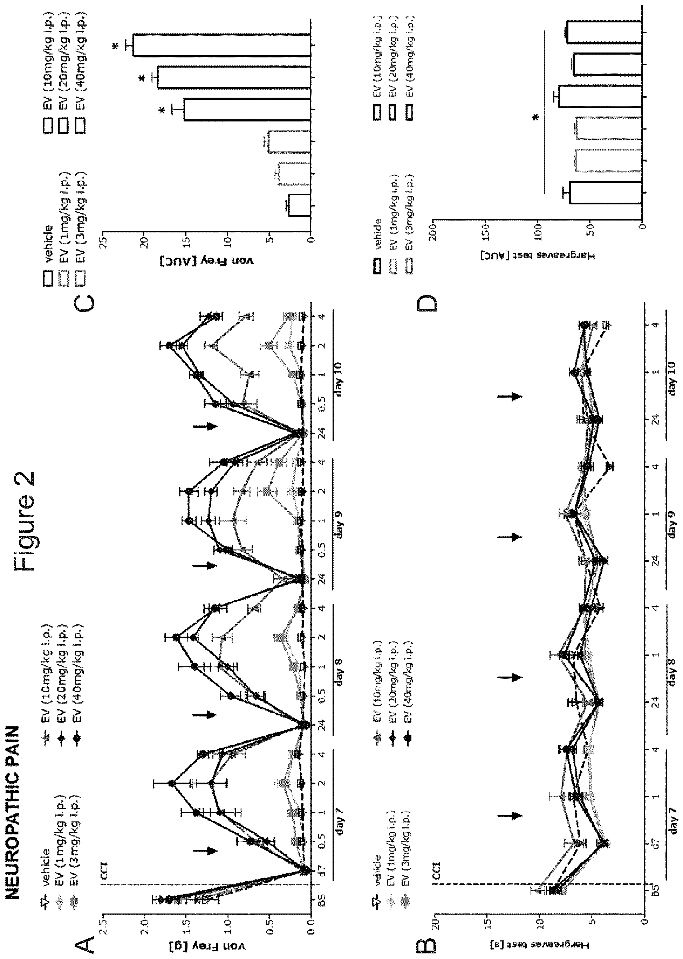

**Figure 2****:** Chronic i.p. administration of EV131 significantly attenuated mechanical hypersensitivity while producing weak effect on thermal hypersensitivity in the chronic constriction injury (CCI) model in mice. (**A-D**) Effects of systemic i.p. injection of EV131 (1, 3, 10, 20 or 40 mg/kg) or vehicle in CCI mice on (**A**) the mechanical withdrawal threshold measured with von Frey filaments and (**B**) the thermal withdrawal threshold measured with Hargreaves test. The measurements were assessed before injury as basal pain threshold (BS) and then 7 days following the injury (d7). The effect of EV131 was assessed 0.5 h, 1 h, 2 h, 4 h and 24 h after each of four consecutive i.p. injections. The arrow indicates each of four i.p. injections of EV131. Data are presented as means ± S.E.M, n = 6-14 in each group. (**C**) The area under the curve (AUC) summarizing measurements in A. F(5,32)=102.2 P<0.0001 vs. vehicle control animals (one-way ANOVA, followed by Bonferroni's comparison post-hoc test) (**D**) The area under the curve (AUC) summarizing measurements in B. F(5,32)=2.81 P=0.03 vs. vehicle control animals (one-way ANOVA, followed by non-significant Bonferroni's comparison post-hoc test).

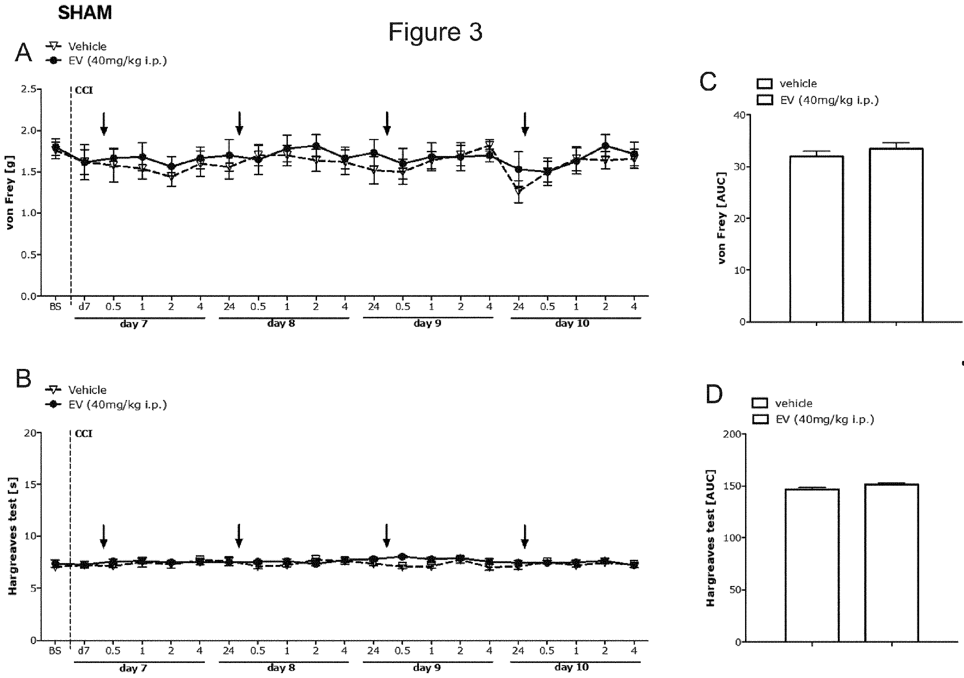

**Figure 3****:** Chronic i.p. administration of EV131 did not attenuate mechanical hypersensitivity or thermal hypersensitivity in sham mice. (**A-D**) Effects of systemic i.p. injection of EV131 (40 mg/kg) or vehicle in sham mice on (**A**) the mechanical withdrawal threshold measured with von Frey filaments and (**B**) the thermal withdrawal threshold measured with Hargreaves test. The measurements were assessed before injury as basal pain threshold (BS) and then 7 days following the injury (d7). The effect of EV131 was assessed 0.5 h, 1 h, 2 h, 4 h and 24 h after each of four consecutive i.p. injections. The arrow indicates each of four i.p. injections of EV131. Data are presented as means ± S.E.M, n = 5-6 in each group. (**C**) The area under the curve (AUC) summarizing measurements in A. t11=0.85 P=0.41 vs. vehicle control animals (unpaired t-test) (**D**) The area under the curve (AUC) summarizing measurements in B. t11=1.799 P=0.09 vs. vehicle control animals (unpaired t-test).

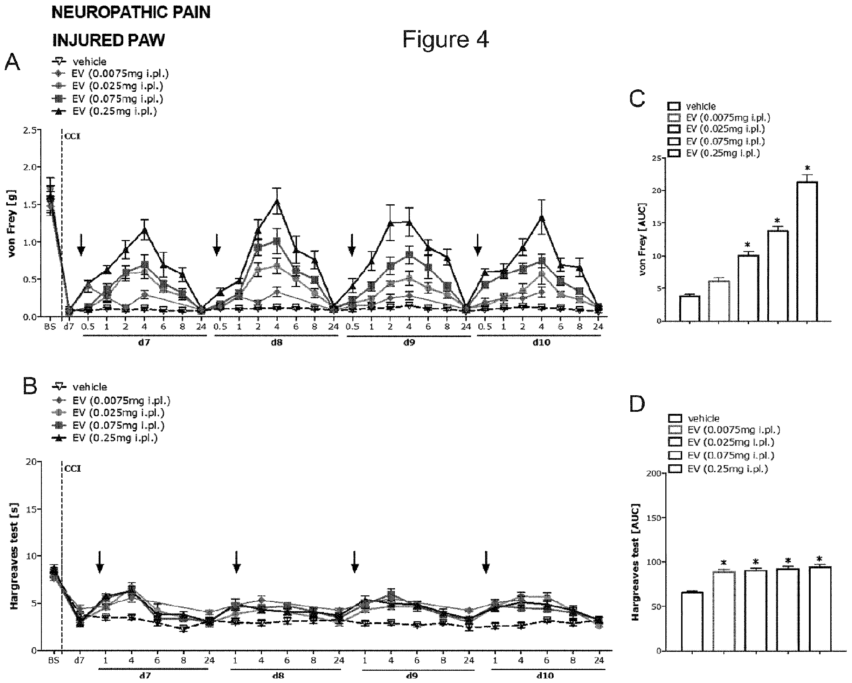

**Figure 4****:** Chronic localized i.pl. (intraplantar) administration of EV131 into the plantar surface of the injured paw significantly attenuated mechanical hypersensitivity while producing weak effect on thermal hypersensitivity in the chronic constriction injury (CCI) model in mice. (**A-D**) Effects of localized i.pl. injection of EV131 (0.0075, 0.025, 0.075, 0.25 mg/50µl; 50µl/injection) or vehicle in CCI mice on (**A**) the mechanical withdrawal threshold measured with von Frey filaments and (**B**) the thermal withdrawal threshold measured with Hargreaves test. The measurements were assessed before injury as basal pain threshold (BS) and then 7 days following the injury (d7). The effect of EV131 was assessed 0.5 h, 1 h, 2 h, 4 h, 6 h, 8 h and 24 h after each of four consecutive i.p. injections. The arrow indicates each of four i.pl. injections of EV131. Data are presented as means ± S.E.M, n = 6 in each group. (**C**) The area under the curve (AUC) summarizing measurements in A. F(4,25)=93.38 P<0.0001 vs. vehicle control animals (one-way ANOVA, followed by Bonferroni's comparison post-hoc test) (**D**) The area under the curve (AUC) summarizing measurements in B. F(4,25)=17.11 P<0.0001 vs. vehicle control animals (one-way ANOVA, followed by Bonferroni's comparison post-hoc test).

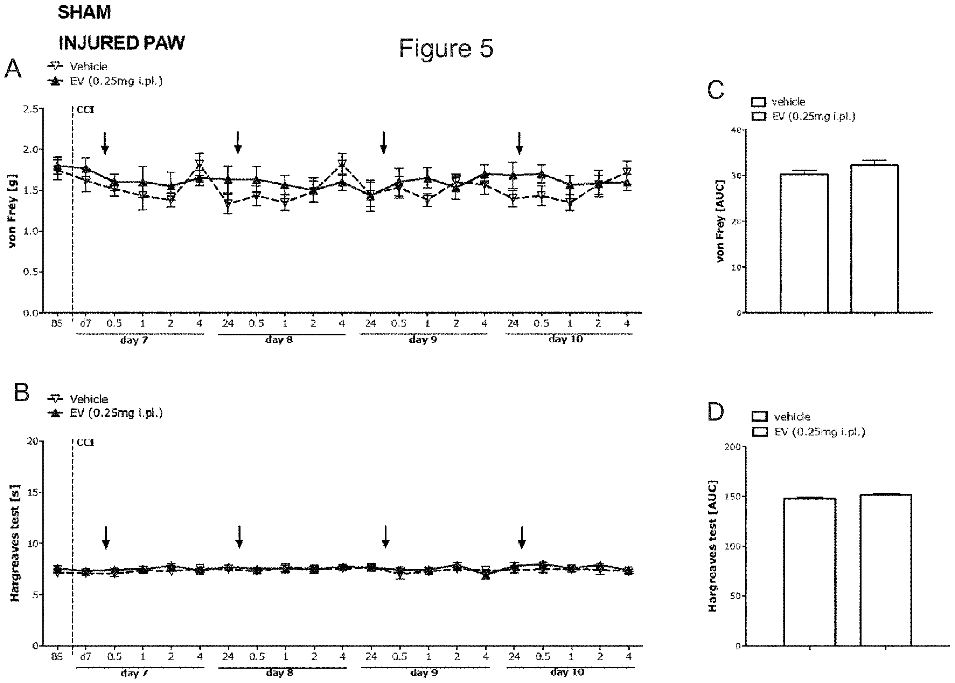

**Figure 5****:** Chronic localized i.pl. administration of EV131 into the plantar surface of the injured paw did not attenuate mechanical hypersensitivity or thermal hypersensitivity in sham mice. (**A-D**) Effects of localized i.pl. injection of EV131 (0.25 mg/50µl; 50µl/injection) or vehicle in sham mice on (**A**) the mechanical withdrawal threshold measured with von Frey filaments and (**B**) the thermal withdrawal threshold measured with Hargreaves test. The measurements were assessed before injury as basal pain threshold (BS) and then 7 days following the injury (d7). The effect of EV131 was assessed 0.5 h, 1 h, 2 h, 4 h and 24 h after each of four consecutive i.pl. injections. The arrow indicates each of four i.p. injections of EV131. Data are presented as means ± S.E.M, n = 6 in each group. **C**) The area under the curve (AUC) summarizing measurements in A. t11=1.31 P=0.22 vs. vehicle control animals (unpaired t-test) (**D**) The area under the curve (AUC) summarizing measurements in B. t11=1.44 P=0.18 vs. vehicle control animals (unpaired t-test).
**Figure 6****:** Primary sequence of the FS-HBP2 protein, including the signal sequence, aligned with the encoding DNA sequence. The end of the signal sequence is indicated by the arrow.

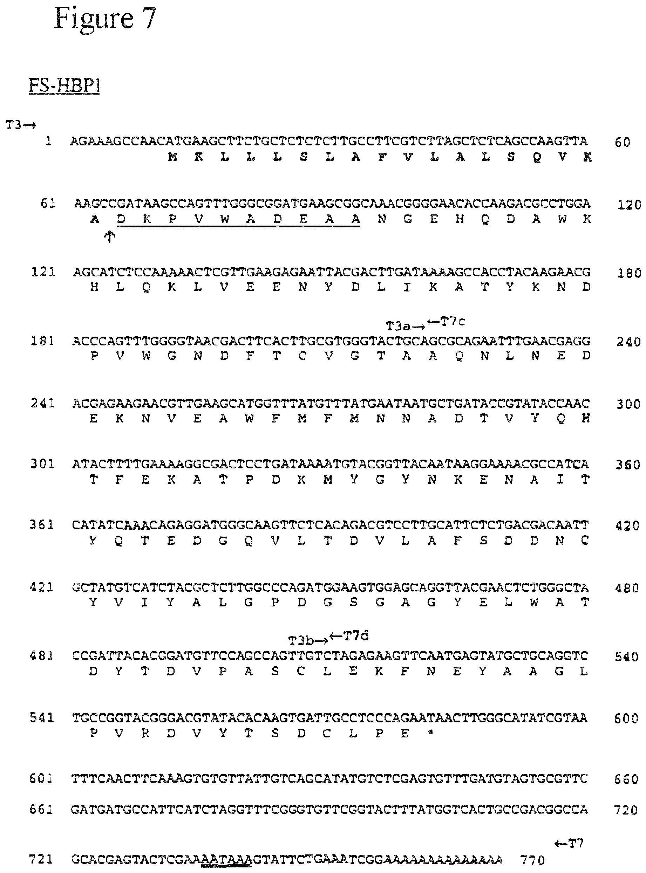

**Figure 7****:** Primary sequence of the FS-HBP1 protein, including the signal sequence, aligned with the encoding DNA sequence. The end of the signal sequence is indicated by the arrow.

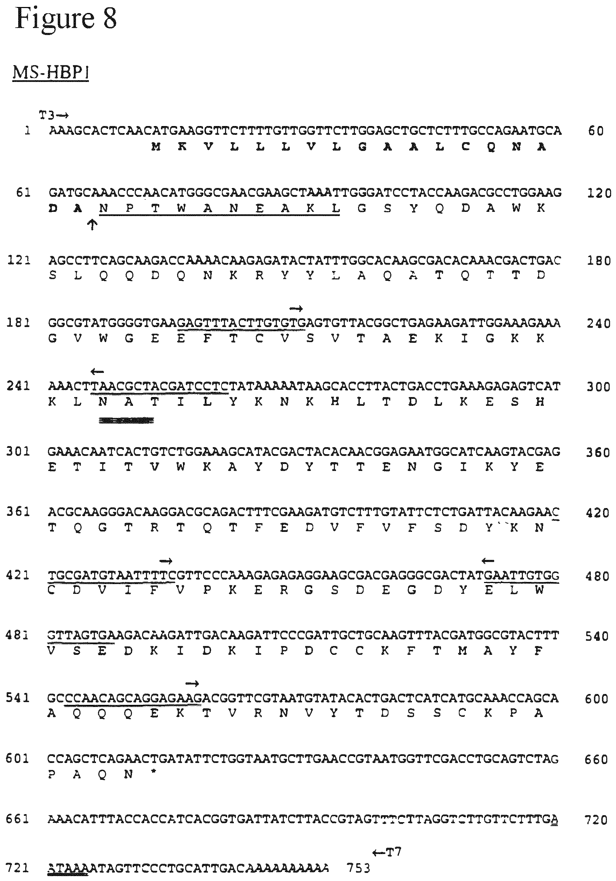

**Figure 8****:** Primary sequence of the MS-HBP1 protein, including the signal sequence, aligned with the encoding DNA sequence. The end of the signal sequence is indicated by the arrow.

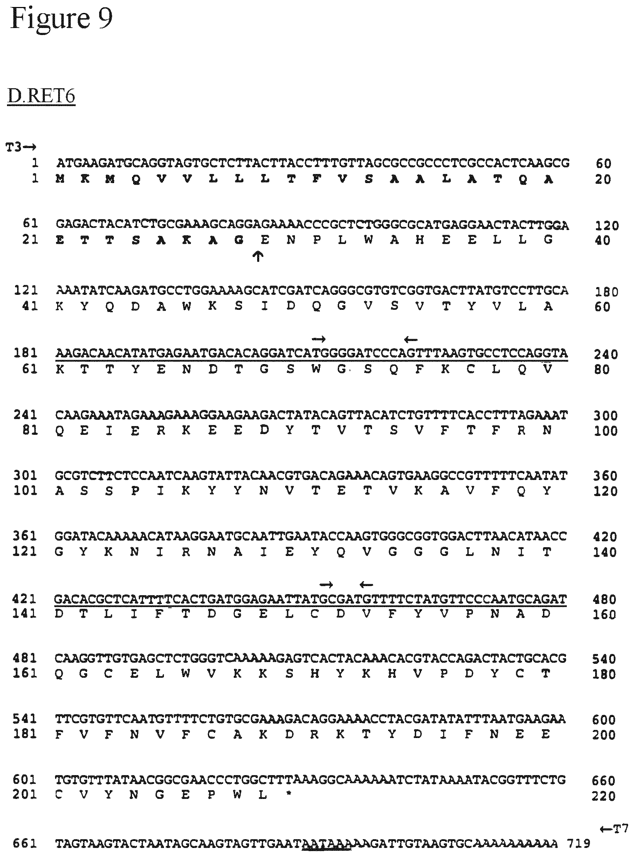

**Figure 9****:** Primary sequence of the D.RET6 protein, including the signal sequence, aligned with the encoding DNA sequence. The end of the signal sequence is indicated by the arrow.

### Examples

### Example 1: Assessment of the efficacy of recombinant histamine binding protein EV131 in neuropathic pain

The native protein EV131 occurs in tick saliva where its anti-inflammatory activity counters the host's immunological response at the tick feeding site.

Methods: Neuropathic pain in adult male C57BL/6J (B6) mice (n=6/group) was induced by chronic constriction injury (CCI) of the sciatic nerve. The effect of EV131 (1, 3 and 10 mg/kg i.p.) on mechanical and thermal hypersensitivity was determined 0.5, 1 and 24 h after EV131 (4 injections every 24 h for 4 days) and compared to saline controls. Mechanical and thermal hypersensitivity was assessed using von Frey filaments and Hargreaves' tests.

Results: In neuropathic mice, EV131 at the dose of 10 mg/kg, but not 1 and 3 mg/kg, reduced mechanical hypersensitivity (AUC [g days] - 10 mg: 10.47±1.75[SEM], 3 mg: 4.41±1.23[SEM], 1 mg: 4.03±1.37[SEM], saline: 2.22±1.21[SEM]). EV131 did not affect thermal hypersensitivity. The results are shown in Figure 1.

Conclusions: Our findings provide the first evidence for the analgesic effects of EV131 upon neuropathic pain related mechanical hypersensitivity and thus emphasize the importance of the histamine system in the modulation of peripheral neuropathies.

### Example 2: Assessment of the efficacy of recombinant histamine binding protein EV131 in neuropathic pain

### Subjects

*C57BL*/*6J mice.* Adult male C57BL/6J mice (8 weeks of age; 20-25 g; Charles River Laboratories, Kent, UK) were allowed to acclimate to the colony room (Durham University, UK or Comparative Biology Centre, Newcastle University, UK) for at least 7 days after arrival and were housed in polyethylene cages (4 per cage), controlled for temperature (21°C) and humidity (55%) under a regular 12-h day/night cycle (lights on at 8:00 A.M.; lights off at 8:00 P.M.). Standard laboratory rodent chow and water were available *ad libitum.* Animals were habituated to testing procedures for at least 3-4 days before experiments. The handling and testing of the animals were conducted during the light phase, between 9:00 A.M. and 6:00 P.M. All efforts were made to minimize animal suffering and to reduce the number of animals used in the study. Experimental protocols were performed under UK Home Office licence, with local ethical approval, and in accordance with current UK legislation as defined in the Animals (Scientific Procedures) Act 1986. The ARRIVE guidelines have been followed in reporting this study.

### Drugs: preparation and administration

*Systemic injections.* For systemic (i.p.) admistration EV131 (SEQ ID NO: 10) was prepared in phosphate-buffered saline as a stock solution of 5.8 mg/mL and then prepared immediately before injections in a vehicle (saline) solution at required concentrations. Mice were weighed and injected intraperitoneally (i.p.) with EV131 (1-40 mg per kg body weight) or equivalent vehicle (saline) solution without EV131 as a control group. EV131/vehicle was injected four times (every 24 h for four days).

*Intraplantar injections.* For intraplantar (i.pl.) administration, EV131 (SEQ ID NO: 10; 0.0075-0.25 mg per 50 µl; 50 µl per one injection) was prepared from a stock solution of 5.8 mg/mL immediately before injections in a vehicle (saline) solution. Intraplantar (i.pl.) injections were given over 1 min in a volume of 50 µl without anesthesia into the plantar surface of the animal hind paw ipsilateral to sciatic nerve injury. Control animals received 50 µl of equivalent vehicle solution without EV131. EV131/vehicle was injected four times (every 24 h for four days).

### Pain model

*Sciatic nerve injury.* Mice were subjected to peripheral neuropathy induced by chronic constriction injury (CCI) of the sciatic nerve as described by Bennett and Xie [18], with slight modifications for mice [19,20]. The sciatic nerve injury was performed under isoflurane anesthesia delivered via a nose cone (2% isoflurane with oxygen as the carrier gas). The skin was shaved and an incision was made just below the right hipbone, parallel to the sciatic nerve. The biceps femoris and the gluteus superficialis were separated, and the right sciatic nerve was exposed. Proximal to the sciatic trifurcation, the injury was produced by three loose ligations around the sciatic nerve. The ligatures (4/0 silk) were tied loosely around the nerve with 1 mm spacing, until they elicit a brief twitch in the respective hindlimb, which prevented from applying a too strong ligation. The total length of nerve affected was 3-4 mm. Muscle and skin were closed in two separate layers. For sham surgery, the sciatic nerve was exposed as described above but no contact was made with the nerve. Both mechanical threshold at the lateral plantar surface of the hind paw and thermal sensitivity were assessed before nerve injury (as basal pain threshold) and then testing began on day 7 after surgery and continued for 3 days post surgery (total of 4 days of behavioural testing).

### Behavioural testing

In all experiments the observer was not aware of the dose of EV131 given in the intraplantar/intraperitoneal injections. Each animal was subjected to von Frey testing followed by the Hargreaves test.

*Mechanical stimulation.* Mechanical sensitivity was assessed by measuring the withdrawal threshold of the paw ipsilateral to the site of injury in response to mechanical stimuli using von Frey filaments (Ugo Basile, Gemonio, Italy). Animals were placed in a plastic cage with a wire net floor and were allowed to habituate before testing began. Animals were also habituated over a period of 3-4 consecutive days by recording a series of baseline measurements. The filaments were applied in ascending order, each five times at an interval of 2-3 seconds to the plantar surface of the hind paw as described previously [21,22] and the smallest filament eliciting a foot withdrawal response was considered the threshold stimulus. The strength of the von Frey stimuli used in the present experiments ranged from 0.04 to 6.0g.

*Thermal stimulation.* Mice were tested for the latency of paw withdrawal response to a noxious thermal stimulus using a radiant heat-emitting device (IITC Life Science Inc., USA) as described by [23]. Mice were placed individually into Plexiglass chambers on an elevated glass platform and allowed to habituate to the apparatus before testing began. Animals were also habituated over a period of 3-4 consecutive days by recording series of baseline measurements. A radiant heat source of constant intensity was applied to the plantar surface of the paw through the glass plate and the latency to paw withdrawal was measured. Hind paw received 3 stimuli and the inter-stimulus interval was at least 3 min to prevent injury. Withdrawal latencies were defined as the mean of readings for hind paw. A cutoff of 20 s was employed to avoid tissue injury.

### Statistical Data Analysis

Data analysis and statistical comparisons were performed using GraphPad PrismTM, version 7.00 for Windows, GraphPad Software, CA, USA, www.graphpad.com.

Results are presented in the graphs as mean ± SEM. Each group included 5-14 animals. Statistical analysis was performed by one- or two-way analyses of variance (ANOVA) with Bonferroni's multiple comparison post-hoc tests or by unpaired Student's t-test when two groups were compared. A value of p < 0.05 was considered to be statistically significant.

### Results

In neuropathic mice subjected to chronic constriction injury (CCI) model of neuropathic mice, EV131 when injected i.p. at the dose of 10, 20 and 40 mg/kg, but not 1 and 3 mg/kg, significantly reduced mechanical hypersensitivity (AUC [g·days] - 40 mg: 21.3±0.9[SEM], 20 mg: 18.3±0.7[SEM], 10 mg: 15.2±1.4[SEM], 3 mg: 5.1±0.4[SEM], 1 mg: 3.9±0.4[SEM], saline: 2.7±0.3[SEM]; F(5,32)=102.2, P<0.0001; see Figure 2A and 2C), while only mildly affected thermal hypersensitivity (AUC [g days] - 40 mg: 71.2±2.5[SEM], 20 mg: 65.1±2.1[SEM], 10 mg: 79.1±5.4[SEM], 3 mg: 62.6±1.7[SEM], 1 mg: 63.0±1.4[SEM], saline: 69.1±6.3[SEM]; F(5,32)=2.8, P=0.03; Bonferroni's multiple comparison test non-significant; see Figure 2B and 2D). Interestingly, EV-131 when injected i.pl. at the lower dose range than compared to i.p. treatment also produced analgesic effect observed in mechanical hypersensitivity testing (AUC [g·days] - 0.25 mg: 21.2±1.1[SEM], 0.075 mg: 13.8±0.7[SEM], 0.025 mg: 10.1±0.6[SEM], 0.0075 mg: 6.1±0.5[SEM], saline: 3.8±0.2[SEM]; F(4,25)=93.4, P<0.0001; see Figure 4A and 4C) and only moderately affected thermal hypersensitivity (AUC [g days] - 0.25 mg: 94.2±2.9[SEM], 0.075 mg: 92.2±2.7[SEM], 0.025 mg: 89.9±2.8[SEM], 0.0075 mg: 88.3±3.6[SEM], saline: 65.2±2.0[SEM]; F(4,25)=17.1, P<0.0001; see Figure 4B and 4D). Both systemic i.p. and local i.pl. administration of EV-131 did not affect pain threshold measured in sham animals (P>0.05; see Figure 3 and 5) or when assessed on the uninjured paw (P>0.05).

**TABLE 1**

| SEQUENCE COMPARISON OF FS-HBP1 (top line), FS-HBP2 (middle line) and MS-HBP1 (bottom line). Identical residues are marked "=" below the three lines of sequence. | | | | | | | | | | | | | | | | | | | |
|---|---|---|---|---|---|---|---|---|---|---|---|---|---|---|---|---|---|---|---|
| The sequences were aligned as described above | | | | | | | | | | | | | | | | | | | |
| D | K | P | V | W | A | D | E | A | A | N | G | E | H | Q | D | A | w | K | H |
| N | Q | P | D | W | A | D | E | A | A | N | G | A | H | Q | D | A | W | K | S |
| N | | P | T | W | A | N | E | A | K | L | G | S | Y | Q | D | A | W | K | S |
| | | = | | = | = | | = | = | | | = | | | = | = | = | = | = | |
| | | | | | | | | | | | | | | | | | | | |
| L | Q | K | L | V | E | E | N | | | Y | D | L | I | K | A | T | Y | K | N |
| L | K | A | D | V | | E | N | V | | Y | Y | M | V | K | A | T | Y | K | N |
| L | Q | Q | | | D | Q | N | K | R | Y | Y | L | A | Q | A | T | Q | T | T |
| = | | | | | | | = | | | = | | | | | = | = | | | |
| | | | | | | | | | | | | | | | | | | | |
| D | P | V | W | G | N | D | F | T | C | V | G | T | A | A | Q | N | L | N | E |
| D | P | V | w | G | N | D | F | T | C | V | G | V | M | A | N | D | V | N | E |
| D | G | V | W | G | E | E | F | T | C | V | S | V | T | A | E | K | I | G | |
| = | | = | = | = | | | = | = | = | = | | | | = | | | | | |
| | | | | | | | | | | | | | | | | | | | |
| D | E | K | N | V | E | A | W | F | M | F | M | N | N | A | D | T | V | Y | Q |
| D | E | K | S | I | Q | A | E | F | L | F | M | N | N | A | D | T | N | M | Q |
| | K | K | K | L | N | A | T | I | L | Y | K | N | K | H | L | T | D | L | K |
| | | = | | | | = | | | | | | = | | | | = | | | |
| | | | | | | | | | | | | | | | | | | | |
| H | T | F | E | K | A | T | P | D | K | M | Y | G | Y | N | K | E | N | A | I |
| F | A | T | E | K | V | T | A | V | K | M | Y | G | Y | N | R | E | N | A | F |
| E | S | H | E | T | I | T | V | W | K | A | Y | D | Y | T | T | E | N | G | I |
| | | | = | | | = | | | = | | = | | = | | | = | = | | |
| | | | | | | | | | | | | | | | | | | | |
| T | Y | Q | T | E | D | G | | | | Q | V | L | T | D | V | L | A | F | S |
| R | Y | E | T | E | D | G | | | | Q | V | F | T | D | V | I | A | Y | S |
| K | Y | E | T | Q | | G | T | R | T | Q | T | F | E | D | V | F | V | F | S |
| | = | | = | | | = | | | | = | | | | = | = | | | | = |
| | | | | | | | | | | | | | | | | | | | |
| D | | D | N | C | Y | V | I | Y | A | L | G | P | D | G | S | G | A | G | |
| A | | D | N | C | D | V | I | Y | V | P | G | T | D | G | N | E | E | G | |
| D | Y | K | N | C | D | V | I | F | V | P | K | E | R | G | S | D | E | G | D |
| | | | | | | | | | | | | | | | | | | | |
| | | | | | | | | | | | | | | | | | | | |
| Y | E | L | W | A | T | | D | | Y | T | D | V | P | A | S | C | L | E | K |
| Y | E | L | W | T | T | | D | | Y | D | N | I | P | A | N | C | L | N | K |
| Y | E | L | W | V | S | E | D | K | I | D | K | I | P | | D | C | C | | K |
| | | | | | | | | | | | | | | | | | | | |
| | | | | | | | | | | | | | | | | | | | |
| F | N | | E | Y | | A | A | G | L | P | | | V | R | D | V | Y | T | |
| F | N | | E | Y | | A | V | G | R | E | | | T | R | D | V | F | T | |
| F | T | M | A | Y | F | A | Q | Q | Q | E | K | T | V | R | N | V | Y | T | D |
| = | | | | = | | = | | | | | | | | = | | = | | = | |
| | | | | | | | | | | | | | | | | | | | |
| S | D | C | L | P | | | | | E | | | | | | | | | | |
| S | A | C | L | | | | | | E | | | | | | | | | | |
| S | S | C | K | P | A | P | A | Q | N | | | | | | | | | | |
| = | | = | | | | | | | | | | | | | | | | | |
| | | | | | | | | | | | | | | | | | | | |

### References

¹ Colloca, L. et al. Nature Reviews, Disease primers, Neuropathic pain, 2017, vol. 3, 17002, 1-19;
² Brain and Spine Foundation "What causes neuropathic pain?" - https://www.brainandspine.org.uk/neuropathic-pain;
³ Finnerup, N.B. et al. and Attal, N. Pharmacotherapy of neuropathic pain: time to rewrite the rulebook? Pain Manag. 6, 1-3, 2016;
⁴ Panula P, Chazot PL, Cowart M, Gutzmer R, Leurs R, Liu WL, Stark H, Thurmond RL, Haas HL., International Union of Basic and Clinical Pharmacology. XCVIII. Histamine Receptors. Pharmacol Rev. 2015 Jul;67(3):601-55. doi: 10.1124/pr.114.010249.
⁵ Obara, I., Rosa, A., Battle, E., Pini, A., Liu, W. L. and Chazot, P. BPS 2016. OB056 Novel peripheral histamine H3 receptor antagonist ZPL868087 attenuates mechanical hypersensitivity in neuropathic pain in mice;
⁶ Weston-Davies Wet al. (2005). Ann NY Acad Sci. 1056:189-196;
⁷ WO 97/44451;
⁸ WO 2009/141621;
⁹ WO 2004/087188;
¹⁰ K. Sunil kumar Reddy, M. U. R. Naidu, P. Usha Rani, and T. Ramesh Kumar Rao, Human experimental pain models: A review of standardized methods in drug development., J Res Med Sci. 2012 Jun; 17(6): 587-595. PMCID: PMC3634303
¹¹ Needleman & Wunsch (1970) J. Mol. Biol. 48, 443-453
¹² Rice et al. (2000) Trends Genet 16:276-277
¹³ Rzodkiewicz P, Gasinska E, Maslinski S, Bujalska-Zadrozny M, Antinociceptive properties of esculetin in non-inflammatory and inflammatory models of pain in rats., Clin Exp Pharmacol Physiol. 2015 Feb;42(2):213-9. doi: 10.1111/1440-1681.12346.
¹⁴ Berkner, K.L., in Curr. Top. Microbiol. Immunol., 158, 39-66 (1992);
¹⁵ Muzyczka, N., in Curr. Top. Microbiol. Immunol, 158, 97-129 (1992);
¹⁶ U.S. Patent No. 5,252,479;
¹⁷ see Chapter 20, Gene Therapy and other Molecular Genetic-based Therapeutic Approaches, (and references cited therein) in Human Molecular Genetics (1996), T Strachan and A P Read, BIOS Scientific Publishers Ltd
¹⁸ Bennett GJ, Xie YK. A peripheral mononeuropathy in rat that produces disorders of pain sensation like those seen in man. Pain, 1988, 33:87-107.
¹⁹ Obara I, Mika J, Schafer MK, Przewlocka B. Antagonists of the kappa-opioid receptor enhance allodynia in rats and mice after sciatic nerve ligation. Br J Pharmacol, 2003, 140: 538-546.
²⁰ Osikowicz M, Mika J, Makuch W, Przewlocka, B. Glutamate receptor ligands attenuate allodynia and hyperalgesia and potentiate morphine effects in a mouse model of neuropathic pain. Pain, 2008, 139: 117-126.
²¹ Bourquin AF, Suveges M, Pertin M, Gilliard N, Sardy S, Davison AC, Spahn DR, Decosterd I. Assessment and analysis of mechanical allodynia-like behavior induced by spared nerve injury (SNI) in the mouse. Pain, 2006, 122:14.
²² Obara I, Tochiki KK, Géranton SM, Carr FB, Lumb BM, Liu Q, Hunt SP. Systemic inhibition of the mammalian target of rapamycin (mTOR) pathway reduces neuropathic pain in mice. Pain, 2011, 152:2582-2595.
²³ Hargreaves K, Dubner R, Brown F, Flores C, Joris J. A new and sensitive method for measuring thermal nociception in cutaneous hyperalgesia. Pain, 1988, 32:77-88.

## Claims

1. An histamine binding protein (HBP) for use in a method of treating neuropathic pain.

2. An HBP for use of claim 1, which is derivable from the saliva of a tick.

3. An HBP for use of any one of the preceding claims, wherein the HBP is selected from FS-HBP2, FS-HBP1, MS-HBP1 and D.RET6, such as wherein the HBP is FS-HBP2, optionally wherein the HBP comprises or consists of the amino acid sequence provided in SEQ ID NO:2.

4. An HBP for use according to any one of the preceding claims, which is a variant of an HBP as described in claim 2 or claim 3, wherein the variant has a protein sequence having at least 70% sequence identity to a protein sequence selected from any one of SEQ ID NOs: 2, 4, 6, 8 and 10, and wherein the variant retains the biological function of binding to histamine.

5. An HBP for use according to any one of the preceding claims which is a fragment of a full length HBP or which is a fragment of a variant thereof, wherein the variant has a protein sequence having at least 70% sequence identity to a protein sequence selected from any one of SEQ ID NOs: 2, 4, 6, 8 and 10, and wherein the fragments and the variant retain the biological function of binding to histamine.

6. A HBP for use according to any one of the preceding claims, wherein the HBP is administered at a dosage of more than 3, 5, 6, 7, 8, 9, or 10, 20, 30 or 40mg mg/kg body weight, optionally wherein the dosage is between 2-10 mg/kg, 4-8 mg/kg or is about 6 mg/kg body weight, wherein one to six doses are given every 24 hours.

7. A purified nucleic acid molecule encoding an HBP as described in any one of the preceding claims for use in a method of treating neuropathic pain.

8. A purified nucleic acid molecule for use according to claim 7, which comprises or consists of:
(a) a cDNA sequence encoding the amino acid sequence of SEQ ID NO:2, optionally which comprises or consists of the cDNA sequence of SEQ ID NO:1; or
(b) a cDNA sequence that encodes a variant or active fragment of SEQ ID NO:2 or an active fragment of such a variant, wherein the variant has a protein sequence having at least 70% sequence identity to a protein sequence selected from any one of SEQ ID NOs: 2, 4, 6, 8 and 10, and wherein the variant retains the biological function of binding to histamine.

9. A vector comprising a purified nucleic acid molecule according to claim 7 or claim 8 for use in a method of treating neuropathic pain.

10. A pharmaceutical composition comprising an HBP, a purified nucleic acid or a vector according to any one of the preceding claims, in conjunction with a pharmaceutically-acceptable carrier, for use in a method of treating neuropathic pain.

11. An HBP, purified nucleic acid, vector or pharmaceutical composition for use according to any one of claims 1 to 10, wherein the neuropathic pain is neuropathic pain in which histamine plays a role.

12. An HBP, purified nucleic acid, vector or pharmaceutical composition for use according to any one of claims 1 to 11, wherein the method:
(a) involves preventing histamine from binding to its receptors; and/or
(b) involves treating neuropathic pain that is observed as hypersensitivity to mechanical stimuli, optionally wherein:
(i) the neuropathic pain is allodynia and/or hyperalgesia; and/or
(ii) the treatment results in improvements in the sensations of one or more of touch (including light touch), "pins and needles", numbness, itching and excruciating pain.

13. An HBP, purified nucleic acid, vector or pharmaceutical composition for use according to any one of claims 1 to 12, wherein:
(a) the neuropathic pain is peripheral neuropathic pain, optionally wherein the patient being treated for peripheral neuropathic pain has a condition or disease selected from damaged, irritated or diseased peripheral nerves, peripheral neuropathy, peripheral nerve injury-induced neuropathic pain, diabetes mellitus, diabetic neuropathy, diabetic polyneuropathy, pre-diabetes, metabolic dysfunction, infectious disease (e.g. HIV infection or leprosy), an immune disorder (e.g. Guillain-Barré syndrome), an inflammatory disorder, an inherited neuropathy, an inherited channelopathy (e.g. inherited erythromelalgia), injury to the sciatic nerve, spinal stenosis, trigeminal neuralgia, postherpetic neuralgia, peripheral nerve injury pain, post-amputation pain, painful polyneuropathy, painful radiculopathy, spinal cord injury, post-traumatic neuropathy, postsurgical neuropathy, cervical polyradiculopathy, lumbar polyradiculopathy and complex regional pain syndrome type 2;
(b) the neuropathic pain is central neuropathic pain, optionally wherein the patient being treated for central neuropathic pain has a condition or disease selected from a cerebrovascular disease affecting the central somatosensory pathways, a neurodegenerative disease and a spinal cord lesion or disease; or
(c) the neuropathic pain is a mixture of both peripheral and central, optionally wherein the patient being treated for peripheral and central neuropathic pain has Parkinson's disease.

14. An HBP, purified nucleic acid, vector or pharmaceutical composition for use according to any one of claims 1 to 13, wherein the patient being treated for neuropathic pain is also being treated with chemotherapy or has previously undergone treatment with chemotherapy.

## Patentansprüche

1. Histamin bindendes Protein (HBP) zur Verwendung bei einem Verfahren zur Behandlung neuropathischer Schmerzen.

2. HBP zur Verwendung nach Anspruch 1, das sich aus dem Speichel einer Zecke gewinnen lässt.

3. HBP zur Verwendung nach einem der vorhergehenden Ansprüche, wobei das HBP aus FS-HBP2, FS-HBP1, MS-HBP1 und D.RET6 ausgewählt ist, wie wobei es sich bei dem HBP um FS-HBP2 handelt, gegebenenfalls wobei das HBP die unter SEQ ID NO:2 angegebene Aminosäuresequenz umfasst bzw. daraus besteht.

4. HBP zur Verwendung gemäß einem der vorhergehenden Ansprüche, bei dem es sich um eine Variante eines HBP wie in Anspruch 2 oder Anspruch 3 beschrieben handelt, wobei die Variante eine Proteinsequenz mit einer Sequenzidentität von wenigstens 70 % mit einer aus einer von SEQ ID NO: 2, 4, 6, 8 und 10 ausgewählten Proteinsequenz aufweist und wobei bei der Variante die biologische Funktion der Bindung an Histamin erhalten ist.

5. HBP zur Verwendung gemäß einem der vorhergehenden Ansprüche, bei dem es sich um ein Fragment eines Volllängen-HBP oder ein Fragment einer Variante davon handelt, wobei die Variante eine Proteinsequenz mit einer Sequenzidentität von wenigstens 70 % mit einer aus einer von SEQ ID NO: 2, 4, 6, 8 und 10 ausgewählten Proteinsequenz aufweist und wobei bei den Fragmenten und der Variante die biologische Funktion der Bindung an Histamin jeweils erhalten ist.

6. HBP zur Verwendung gemäß einem der vorhergehenden Ansprüche, wobei das HBP in einer Dosierung von mehr als 3, 5, 6, 7, 8, 9 oder 10, 20, 30 oder 40mg mg/kg Körpergewicht verabreicht wird, gegebenenfalls wobei die Dosierung zwischen 2-10 mg/kg, 4-8 mg/kg oder bei etwa 6 mg/kg Körpergewicht liegt, wobei alle 24 Stunden eine bis sechs Dosen gegeben werden.

7. Gereinigtes Nukleinsäuremolekül, codierend ein HBP wie in einem der vorhergehenden Ansprüche beschrieben, zur Verwendung bei einem Verfahren zur Behandlung neuropathischer Schmerzen.

8. Gereinigtes Nukleinsäuremolekül zur Verwendung gemäß Anspruch 7, das Folgendes umfasst bzw. daraus besteht:
(a) eine cDNA-Sequenz, die die Aminosäuresequenz unter SEQ ID NO:2 codiert, gegebenenfalls das die cDNA-Sequenz unter SEQ ID NO:1 umfasst bzw. daraus besteht; oder
(b) eine cDNA-Sequenz, die eine Variante bzw. ein aktives Fragment von SEQ ID NO:2 oder ein aktives Fragment einer solchen Variante codiert, wobei die Variante eine Proteinsequenz mit einer Sequenzidentität von wenigstens 70 % mit einer aus einer von SEQ ID NO: 2, 4, 6, 8 und 10 ausgewählten Proteinsequenz aufweist und wobei bei der Variante die biologische Funktion der Bindung an Histamin erhalten ist.

9. Vektor, umfassend ein gereinigtes Nukleinsäuremolekül gemäß Anspruch 7 oder Anspruch 8, zur Verwendung bei einem Verfahren zur Behandlung neuropathischer Schmerzen.

10. Pharmazeutische Zusammensetzung, umfassend ein HBP, eine gereinigte Nukleinsäure oder einen Vektor gemäß einem der vorhergehenden Ansprüche in Verbindung mit einem pharmazeutisch unbedenklichen Träger, zur Verwendung bei einem Verfahren zur Behandlung neuropathischer Schmerzen.

11. HBP, gereinigte Nukleinsäure, Vektor oder pharmazeutische Zusammensetzung zur Verwendung gemäß einem der Ansprüche 1 bis 10, wobei es sich bei den neuropathischen Schmerzen um neuropathische Schmerzen handelt, bei denen Histamin eine Rolle spielt.

12. HBP, gereinigte Nukleinsäure, Vektor oder pharmazeutische Zusammensetzung zur Verwendung gemäß einem der Ansprüche 1 bis 11, wobei das Verfahren:
(a) Verhindern der Bindung von involves Histamin an seine Rezeptoren beinhaltet; und/oder
(b) Behandeln neuropathischer Schmerzen beinhaltet, die als Überempfindlichkeit gegenüber mechanischen Reizen beobachtet werden, gegebenenfalls wobei:
(i) es sich bei den neuropathischen Schmerzen um Allodynie und/oder Hyperalgesie handelt; und/oder
(ii) die Behandlung zu Verbesserungen der Empfindungen einer/eines oder mehrerer von Berührung (einschließlich leichter Berührung), "Nadelstichen", Taubheit, Juckreiz und quälenden Schmerzen führt.

13. HBP, gereinigte Nukleinsäure, Vektor oder pharmazeutische Zusammensetzung zur Verwendung gemäß einem der Ansprüche 1 bis 12, wobei:
(a) es sich bei den neuropathischen Schmerzen um periphere neuropathische Schmerzen handelt, gegebenenfalls wobei bei dem gegen periphere neuropathische Schmerzen behandelten Patienten ein Leiden bzw. eine Krankheit vorliegt, das bzw. die aus beschädigten, gereizten oder erkrankten peripheren Nerven, peripherer Neuropathie, durch Verletzung peripherer Nerven induzierten neuropathischen Schmerzen, Diabetes mellitus, diabetischer Neuropathie, diabetischer Polyneuropathie, Prädiabetes, metabolischer Dysfunktion, Infektionskrankheit (z. B. HIV-Infektion oder Lepra), einer Immunkrankheit (z. B. Guillain-Barre-Syndrom), einer Entzündungskrankheit, einer vererbten Neuropathie, einer vererbten Kanalopathie (z. B. vererbte Erythromelalgie), Verletzung des Ischiasnervs, Spinalstenose, Trigeminusneuralgie, postherpetischer Neuralgie, Schmerzen durch periphere Nervenverletzungen, Schmerzen nach Amputation, schmerzhafter Polyneuropathie, schmerzhafter Radikulopathie, Rückenmarkverletzung, posttraumatischer Neuropathie, postoperativer Neuropathie, zervikaler Polyradikulopathie, lumbaler Polyradikulopathie und komplexem regionalem Schmerzsyndrom Typ 2 ausgewählt ist;
(b) es sich bei den neuropathischen Schmerzen um zentrale neuropathische Schmerzen handelt, gegebenenfalls wobei bei dem gegen zentrale neuropathische Schmerzen behandelten Patienten ein Leiden bzw. eine Krankheit vorliegt, das bzw. die aus einer zerebrovaskulären Erkrankung, die die zentralen somatosensorischen Wege betrifft, einer neurodegenerativen Erkrankung und einer Rückenmarksläsion oder -erkrankung ausgewählt ist; oder
(c) es sich um eine Mischung aus sowohl peripheren als auch zentralen neuropathischen Schmerzen handelt, gegebenenfalls wobei bei dem gegen periphere und zentrale neuropathische Schmerzen behandelten Patienten Morbus Parkinson vorliegt.

14. HBP, gereinigte Nukleinsäure, Vektor oder pharmazeutische Zusammensetzung zur Verwendung gemäß einem der Ansprüche 1 bis 13, wobei der gegen neuropathische Schmerzen behandelte Patient auch mit Chemotherapie behandelt wird oder zuvor einer Behandlung mit Chemotherapie unterzogen wurde.

## Revendications

1. Protéine de liaison à l'histamine (HBP) pour une utilisation dans un procédé de traitement d'une douleur neuropathique.

2. HBP pour une utilisation selon la revendication 1, qui peut être dérivée de la salive d'une tique.

3. HBP pour une utilisation selon l'une quelconque des revendications précédentes, la HBP étant choisie parmi FS-HBP2, FS-HBP1, MS-HBP1 et D.RET6, telle que la HBP étant FS-HBP2, éventuellement la HBP comprenant ou étant constituée de la séquence d'acides aminés fournie dans la SEQ ID NO: 2.

4. HBP pour une utilisation selon l'une quelconque des revendications précédentes, qui est un variant d'une HBP telle que décrite dans la revendication 2 ou la revendication 3, le variant ayant une séquence protéique ayant au moins 70 % d'identité de séquence avec une séquence protéique choisie parmi l'une quelconque des SEQ ID NO: 2, 4, 6, 8 et 10, et le variant conservant la fonction biologique de liaison à l'histamine.

5. HBP pour une utilisation selon l'une quelconque des revendications précédentes, qui est un fragment d'une HBP de pleine longueur ou qui est un fragment d'un variant correspondant, le variant ayant une séquence protéique ayant au moins 70 % d'identité de séquence avec une séquence protéique choisie parmi l'une quelconque des SEQ ID NO: 2, 4, 6, 8 et 10, et les fragments et le variant conservant la fonction biologique de liaison à l'histamine.

6. HBP pour une utilisation selon l'une quelconque des revendications précédentes, la HBP étant administrée à un dosage de plus de 3, 5, 6, 7, 8, 9 ou 10, 20, 30 ou 40 mg/kg de poids corporel, éventuellement le dosage étant compris entre 2 et 10 mg/kg, 4 à 8 mg/kg ou étant d'environ 6 mg/kg de poids corporel, une à six doses étant données toutes les 24 heures.

7. Molécule d'acide nucléique purifiée codant pour une HBP telle que décrite dans l'une quelconque des revendications précédentes pour une utilisation dans un procédé de traitement d'une douleur neuropathique.

8. Molécule d'acide nucléique purifiée pour une utilisation selon la revendication 7, qui comprend ou est constituée de :
(a) une séquence d'ADNc codant pour la séquence d'acides aminés de la SEQ ID NO: 2, éventuellement qui comprend ou est constituée de la séquence d'ADNc de la SEQ ID NO: 1 ; ou
(b) une séquence d'ADNc qui code pour un variant ou un fragment actif de la SEQ ID NO: 2 ou un fragment actif d'un tel variant, le variant ayant une séquence protéique ayant au moins 70 % d'identité de séquence avec une séquence protéique choisie parmi l'une quelconque des SEQ ID NO: 2, 4, 6, 8 et 10, et le variant conservant la fonction biologique de liaison à l'histamine.

9. Vecteur comprenant une molécule d'acide nucléique purifié selon la revendication 7 ou la revendication 8 pour une utilisation dans un procédé de traitement d'une douleur neuropathique.

10. Composition pharmaceutique comprenant une HBP, un acide nucléique purifié ou un vecteur selon l'une quelconque des revendications précédentes, en conjonction avec un support pharmaceutiquement acceptable, pour une utilisation dans un procédé de traitement d'une douleur neuropathique.

11. HBP, acide nucléique purifié, vecteur ou composition pharmaceutique pour une utilisation selon l'une quelconque des revendications 1 à 10, la douleur neuropathique étant une douleur neuropathique dans laquelle l'histamine joue un rôle.

12. HBP, acide nucléique purifié, vecteur ou composition pharmaceutique pour une utilisation selon l'une quelconque des revendications 1 à 11, le procédé :
(a) impliquant le fait d'empêcher l'histamine de se lier à ses récepteurs ; et/ou
(b) impliquant le traitement d'une douleur neuropathique qui est observée comme une hypersensibilité à des stimuli mécaniques, éventuellement :
(i) la douleur neuropathique étant l'allodynie et/ou l'hyperalgie ; et/ou
(ii) le traitement entraînant des améliorations dans les sensations d'un ou plusieurs parmi le toucher (y compris le toucher léger), les picotements, l'engourdissement, une démangeaison et une douleur atroce.

13. HBP, acide nucléique purifié, vecteur ou composition pharmaceutique pour une utilisation selon l'une quelconque des revendications 1 à 12,
(a) la douleur neuropathique étant une douleur neuropathique périphérique, éventuellement le patient qui est traité pour une douleur neuropathique périphérique étant atteint d'une affection ou maladie choisie parmi les nerfs périphériques endommagés, irrités ou malades, la neuropathie périphérique, la douleur neuropathique induite par une lésion nerveuse périphérique, le diabète sucré, la neuropathie diabétique, la polyneuropathie diabétique, le prédiabète, un dysfonctionnement métabolique, une maladie infectieuse (p. ex., infection par le VIH ou lèpre), une maladie immunitaire (p. ex., syndrome de Guillain-Barré), un trouble inflammatoire, une neuropathie héréditaire, une canalopathie héréditaire (p. ex., érythrose héréditaire), une lésion du nerf sciatique, une sténose spinale, une névralgie trijumeau, une névralgie postherpétique, une douleur liée aux lésions nerveuses périphériques, une douleur post-amputation, une polyneuropathie douloureuse, une radiculopathie douloureuse, une lésion de la moelle épinière, une neuropathie post-traumatique, une neuropathie postchirurgicale, une polyradiculopathie cervicale, une polyradiculopathie lombaire et le syndrome douloureux régional complexe de type 2 ;
(b) la douleur neuropathique étant une douleur neuropathique centrale, éventuellement le patient qui est traité pour une douleur neuropathique centrale étant atteint d'une affection ou maladie choisie parmi une maladie cérébrovasculaire affectant les voies somatosensorielles centrales, une maladie neurodégénérative et une lésion ou maladie de la moelle épinière ; ou
(c) la douleur neuropathique est un mélange d'à la fois périphérique et centrale, éventuellement le patient qui est traité pour une douleur neuropathique périphérique et centrale étant atteint de la maladie de Parkinson.

14. HBP, acide nucléique purifié, vecteur ou composition pharmaceutique pour une utilisation selon l'une quelconque des revendications 1 à 13, le patient qui est traité pour une douleur neuropathique étant également traité par chimiothérapie ou ayant précédemment subi un traitement par chimiothérapie.
